# EUROPEAN PATENT APPLICATION

(11) **EP 4 151 747 A1**
(43) Date of publication of application: **22.03.2023**
(21) Application number: 21805000.3
(22) Date of filing: 11.05.2021
(51) Int. Cl.: C12Q 1/68, G01N 33/53

(54) **METHOD AND SYSTEM FOR DIAGNOSING CENTRAL NERVOUS SYSTEM DISEASES USING PERIPHERAL BODY FLUIDS WITH MULTI-LABELING BIOMARKERS**

(30) Priority: 11.05.2020 CN 202010390614
(71) Applicant: The First Affiliated Hospital, Zhejiang University School of Medicine, Hangzhou, Zhejiang 310003 (CN); XY Evergreen Technology Company, Beijing 102206 (CN)
(72) Inventor: GUO, Zhen, Hangzhou, Zhejiang 310003 (CN); SONG, Xueru, Hangzhou, Zhejiang 310003 (CN); HAN, Qingqing, Beijing 102206 (CN)
(74) Representative: Lavoix
(86) International application number: PCT/CN2021/092983
(87) International publication number: WO 2021/228067

(57) **Abstract**

The present invention provides a method and system for diagnosing a central nervous system disease, and a composition, and a kit. The diagnosing system comprises: a first detection module, used for detecting a central nervous system-derived marker in a biological body fluid of a subject; a second detection module, used for detecting a central nervous system disease-related marker in the biological body fluid of the subject; and a diagnosis module, diagnosing, on the basis of central nervous system-derived marker and central nervous system disease-related marker detection results respectively obtained by the first detection module and the second detection module, whether the subject suffers from a central nervous system disease.

## Description

### FIELD OF THE INVENTION

The present application relates to the technical field of biomedicine, in particular to the technical field of in vitro diagnostic detection, to a method and system for detection using dual and/or multiple markers, and preferably, a method and system for simultaneous detection using dual and/or multiple markers.

### BACKGROUND OF THE INVENTION

With the development of science and technology, detection of biomarkers plays an increasingly important role in assisting disease diagnosis, differential diagnosis or disease progression monitoring.

Currently, detection of central nervous system disease-associated biomarkers is mainly developed based on detection of cerebrospinal fluid and direct detection of peripheral body fluids. The cerebrospinal fluid-based detection achieves better performance because the cerebrospinal fluid is in direct contact with the brain and the spinal cord and can reflect changes in the central nervous system better. However, the invasive nature of acquisition of cerebrospinal fluid and poor patient acceptance has greatly limited the in-depth development, clinical transformation, and widespread use of biomarkers in the cerebrospinal fluid. The performance of detection using peripheral body fluids is not ideal, and there are two main problems: on the one hand, not all central nervous system-derived components will be detected in peripheral body fluids due to the presence of the blood-brain barrier, even central nervous system-derived biomarkers, especially proteins, which are present in peripheral body fluids have very low abundance; and on the other hand, since many central nervous disease-associated markers are not specifically expressed in the central nervous system and can also be generated in other peripheral organs and/or systems, central nervous system-derived signals are highly susceptible to peripheral components.

Meanwhile, central nervous system diseases have brain region-specific characteristics. For example, the main pathological changes of Alzheimer's disease are atrophy of cerebral cortex such as parietal lobe, temporal lobe, and hippocampus, and Parkinson's disease is mainly caused by insufficient dopamine secretion due to lesions or damage of the nigrostriatal dopamine circuit of basal ganglia. Currently, only brain biopsy can specifically detect and analyze markers in different brain regions. The existing in vitro detection techniques fail to distinguish the sources, and have different diagnosis effects on different diseases. Meanwhile, there is room for improvement in the accuracy of disease diagnosis.

Therefore, how to effectively enrich and specifically label biomarkers capable of reflecting changes in central nervous system diseases in peripheral body fluids is a research focus in the art.

### SUMMARY OF THE INVENTION

As described above, there is no system, method or kit for effectively detecting a central nervous system disease using a peripheral body fluid in the art, and this application is intended to provide a method for effectively identifying different central nervous system-derived markers in a peripheral body fluid, detecting them together with disease-associated markers, and simultaneously detecting multiple markers so as to avoid acquisition of cerebrospinal fluid and reflect changes in the central nervous system through a peripheral body fluid sample.

That is, this application first proposes a concept of diagnosing a central nervous system disease by identifying central nervous system-derived biomarkers in a peripheral body fluid and a detection method.

Specifically, this application relates to the following items.
1. A method for diagnosing a central nervous system disease, comprising:
   detecting a central nervous system-derived marker in a biological fluid of a subject,
   detecting a central nervous system disease-associated marker in the biological fluid of the subject, and
   diagnosing whether the subject suffers from a central nervous system disease based on detection results of the central nervous system-derived marker and the central nervous system disease-associated marker.
2. The method according to item 1, wherein
   the detecting a central nervous system-derived marker in a biological fluid of a subject and the detecting a central nervous system disease-associated marker in the biological fluid of the subject are performed simultaneously.
3. The method according to item 1 or 2, further comprising:
   acquiring the biological fluid of the subject, and pretreating the biological fluid to enrich biological samples in the biological fluid,
   the detecting a central nervous system-derived marker in a biological fluid of a subject referring to detecting a central nervous system-derived marker in enriched biological samples of the subject, and
   the detecting a central nervous system disease-associated marker in the biological fluid of the subject referring to detecting a central nervous system disease-associated marker in the enriched biological samples of the subject.
4. The method according to any one of items 1 to 3, further comprising: counting biological samples in which both of the central nervous system-derived marker and the central nervous system disease-associated marker are detected, and/or
   measuring particle sizes of the biological samples in which both of the central nervous system-derived marker and the central nervous system disease-associated marker are detected, and
   diagnosing whether the subject suffers from a central nervous system disease based on the number of the biological samples and/or the particle sizes of the biological samples.
5. The method according to item 3 or 4, wherein
   the biological samples are extracellular vesicles, and
   preferably, the extracellular vesicles are neuron-derived extracellular vesicles, astrocyte-derived extracellular vesicles, oligodendrocyte-derived extracellular vesicles or microglia-derived extracellular vesicles.
6. The method according to any one of items 1 to 5, wherein the biological fluid is selected from one or more of blood, serum, plasma, saliva, urine, lymph, semen or milk.
7. The method according to any one of items 1 to 6, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.
8. The method according to item 7, wherein
   the cerebrovascular diseases include cerebral infarction, cerebral hemorrhage, cerebral blood supply insufficiency, and transient ischemic attack;
   the spinal cord lesions include acute myelitis, polio, and syringomyelia;
   the central nervous system infections include encephalitis and meningitis;
   the inherited diseases of the nervous system include neurocutaneous syndrome and spinocerebellar ataxia;
   the dysplastic diseases of the nervous system include congenital hydrocephalus and cerebral palsy;
   the autonomic nervous system disease is autonomic nervous dysfunction; and
   the degenerative diseases of the nervous system include degenerative dementia, dyskinetic diseases, and prion diseases.
9. The method according to item 8, wherein
   the degenerative dementia includes Alzheimer's disease (AD), frontotemporal dementia (FTD), Parkinson's disease dementia (PDD), and dementia with Lewy bodies (DLB); and
   the dyskinetic diseases include motor neuron disease (MND), Parkinson's disease (PD), Huntington's disease (HD), Sydenham's chorea, Wilson's disease (WD), multiple system atrophy (MSA), progressive supranuclear palsy (PSP), and corticobasal degeneration (CBD).
10. The method according to any one of items 1 to 9, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.
11. The method according to any one of items 1 to 10, wherein the central nervous system-derived markers and/or the central nervous system disease-associated markers are each selected from one or two or three of proteins, nucleic acids, and lipids.
12. The method according to item 10 or 11, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE),
   preferably, the central nervous system-derived markers are markers located on the surface of central nervous system-derived extracellular vesicles, and
   further preferably, the central nervous system-derived markers are selected from one or more of L1CAM, SHISA9, CNPase, and GLT1 (EAAT2).
13. The method according to item 11, wherein the central nervous system disease-associated markers are proteins selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.
14. The method according to item 11, wherein the central nervous system disease-associated markers are nucleic acids selected from one or more of DNA or RNA serving as biomarkers associated with Alzheimer's disease (AD), and DNA or RNA serving as biomarkers associated with Parkinson's disease (PD) and Parkinson's syndromes (e.g., multiple system atrophy (MSA), progressive supranuclear palsy (PSP), corticobasal degeneration (CBD), and dementia with Lewy bodies (DLB)).
15. The method according to item 14, wherein the DNA or RNA serving as biomarkers associated with AD includes DNA or RNA associated with genes UBAP2L, YBX1, SERF2, UBE2B, RPL10A, H3F3AP4, PPP2CA, NMD3, RNF7, RPLPO, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, or GREB 1.
16. The method according to item 14, wherein the DNA or RNA serving as biomarkers associated with PD includes DNA or RNA associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC100132832, or BLOC1S1-RDH5.
17. The method according to item 3, wherein the method for acquiring the biological fluid of the subject and pretreating the biological fluid to enrich biological samples is selected from one or more of centrifugation, ultracentrifugation, ultrafiltration tube filtration, polymeric sedimentation, and specific antibody capture.
18. The method according to item 11, wherein the central nervous system-derived markers are proteins, and the step of detecting a central nervous system-derived marker in a biological fluid of a subject includes: reacting the biological fluid of the subject, preferably extracellular vesicles of the subject, with a labeled antibody that specifically reacts with central nervous system-derived markers, and detecting the intensity of a labeled signal after the reaction to determine the presence and content of the central nervous system-derived markers.
19. The method according to item 11, wherein the central nervous system disease-associated markers are proteins, and the step of detecting a central nervous system disease-associated marker in the biological fluid of the subject includes: reacting the biological fluid of the subject, preferably extracellular vesicles of the subject, with a labeled antibody that specifically reacts with central nervous system disease-associated markers, and detecting the intensity of a labeled signal after the reaction to determine the presence and content of the central nervous system disease-associated markers.
20. The method according to item 18 or 19, wherein the labeling is selected from one or more of fluorescence labeling, isotope labeling, enzyme labeling, chemiluminescence labeling, quantum dot labeling, and colloidal gold labeling.
21. The method according to item 11, wherein the central nervous system-derived markers are nucleic acids, and the step of detecting a central nervous system-derived marker in a biological fluid of a subject includes: lysing the biological fluid of the subject, preferably extracellular vesicles of the subject, and detecting the presence and content of central nervous system-derived nucleic acids by a DNA or RNA probe or sequencing technique.
22. The method according to item 11, wherein the central nervous system disease-associated markers are nucleic acids, and the step of detecting a central nervous system disease-associated marker in the biological fluid of the subject includes: lysing the biological fluid of the subject, preferably extracellular vesicles of the subject, and detecting the presence and content of central nervous system disease-associated nucleic acids by a DNA or RNA probe or sequencing technique.
23. A system for diagnosing a central nervous system disease, comprising:
   a first detection module, configured to detect a central nervous system-derived marker in a biological fluid of a subject,
   a second detection module, configured to detect a central nervous system disease-associated marker in the biological fluid of the subject, and
   a diagnosis module, configured to diagnose whether the subject suffers from a central nervous system disease based on detection results of the central nervous system-derived markers and the central nervous system disease-associated markers respectively obtained by the first detection module and the second detection module.
24. The system according to item 23, wherein
   the first detection module and the second detection module simultaneously detect the central nervous system-derived marker and the central nervous system disease-associated marker in the biological fluid of the subject; or
   the first detection module and the second detection module are the same module configured to simultaneously detect the central nervous system-derived marker and the central nervous system disease-associated marker in the biological fluid of the subject.
25. The system according to item 23 or 24, further comprising:
   an enrichment module, configured to acquire the biological fluid of the subject and pretreat the biological fluid to enrich biological samples in the biological fluid,
   wherein the first detection module and the second detection module detect the central nervous system-derived marker and the central nervous system disease-associated marker in enriched biological samples of the subject.
26. The system according to any one of items 23 to 25, further comprising:
   a counting module, configured to count biological samples in which both of the central nervous system-derived marker and the central nervous system disease-associated marker are detected, and/or
   a particle size measurement module, configured to measure particle sizes of the biological samples in which both of the central nervous system-derived marker and the central nervous system disease-associated marker are detected, and
   the diagnosis module, configured to diagnose whether the subject suffers from a central nervous system disease based on the number of the biological samples and/or the particle sizes of the biological samples.
27. The system according to item 25 or 26, wherein
   the biological samples are extracellular vesicles, and
   preferably, the extracellular vesicles are neuron-derived extracellular vesicles, astrocyte-derived extracellular vesicles, oligodendrocyte-derived extracellular vesicles or microglia-derived extracellular vesicles.
28. The system according to any one of items 23 to 27, wherein the biological fluid is selected from one or more of blood, serum, plasma, saliva, urine, lymph, semen, and milk.
29. The system according to any one of items 23 to 28, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.
30. The system according to item 29, wherein
   the cerebrovascular diseases include cerebral infarction, cerebral hemorrhage, cerebral blood supply insufficiency, and transient ischemic attack;
   the spinal cord lesions include acute myelitis, polio, and syringomyelia;
   the central nervous system infections include encephalitis and meningitis;
   the inherited diseases of the nervous system include neurocutaneous syndrome and spinocerebellar ataxia;
   the dysplastic diseases of the nervous system include congenital hydrocephalus and cerebral palsy;
   the autonomic nervous system disease is autonomic nervous dysfunction; and
   the degenerative diseases of the nervous system include degenerative dementia, dyskinetic diseases, and prion diseases.
31. The system according to item 30, wherein
   the degenerative dementia includes Alzheimer's disease (AD), frontotemporal dementia (FTD), Parkinson's disease dementia (PDD), and dementia with Lewy bodies (DLB); and
   the dyskinetic diseases include motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).
32. The system according to any one of items 23 to 31, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.
33. The system according to any one of items 23 to 32, wherein the central nervous system-derived markers and/or the central nervous system disease-associated markers are each selected from one or two or three of proteins, nucleic acids, and lipids.
34. The system according to item 32 or 33, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE),
   preferably, the central nervous system-derived markers are markers located on the surface of central nervous system-derived extracellular vesicles, and
   further preferably, the central nervous system-derived markers are selected from one or more of L1CAM, SHISA9, CNPase, and GLT1 (EAAT2).
35. The system according to item 34, wherein the central nervous system disease-associated markers are proteins selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.
36. The system according to item 34, wherein the central nervous system disease-associated markers are nucleic acids selected from one or more of DNA or RNA serving as biomarkers associated with Alzheimer's disease (AD), and DNA or RNA serving as biomarkers associated with Parkinson's disease (PD) and Parkinson's syndromes (e.g., multiple system atrophy (MSA), progressive supranuclear palsy (PSP), corticobasal degeneration (CBD), and dementia with Lewy bodies (DLB)).
37. The system according to item 36, wherein the DNA or RNA serving as biomarkers associated with AD includes DNA or RNA associated with genes UBAP2L, YBX1, SERF2, UBE2B, RPL10A, H3F3AP4, PPP2CA, NMD3, RNF7, RPLPO, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, or GREB 1.
38. The system according to item 36, wherein the DNA or RNA serving as biomarkers associated with PD includes DNA or RNA associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC 100132832, or BLOC 1S 1-RDH5.
39. The system according to item 25, wherein the enrichment module includes a sub-module configured to perform one or more of the following steps: centrifugation, ultracentrifugation, ultrafiltration tube filtration, polymeric sedimentation, and specific antibody capture.
40. The system according to item 33, wherein the central nervous system-derived markers are proteins, and the first detection module is configured to react the biological fluid of the subject, preferably extracellular vesicles of the subject, with a labeled antibody that specifically reacts with central nervous system-derived markers, and detect the intensity of a labeled signal after the reaction to determine the presence and content of the central nervous system-derived markers.
41. The system according to item 33, wherein the central nervous system disease-associated markers are proteins, and the second detection module is configured to react the biological fluid of the subject, preferably extracellular vesicles of the subject, with a labeled antibody that specifically reacts with central nervous system disease-associated markers, and detect the intensity of a labeled signal after the reaction to determine the presence and content of the central nervous system disease-associated markers.
42. The system according to item 40 or 41, wherein the labeling is selected from one or more of fluorescence labeling, isotope labeling, enzyme labeling, chemiluminescence labeling, quantum dot labeling, and colloidal gold labeling.
43. The system according to item 33, wherein the central nervous system-derived markers are nucleic acids, and the first detection module is configured to lyse the biological fluid of the subject, preferably extracellular vesicles of the subject, and detect the presence and content of central nervous system-derived nucleic acids by a DNA or RNA probe or sequencing technique.
44. The system according to item 33, wherein the central nervous system disease-associated markers are nucleic acids, and the second detection module is configured to lyse the biological fluid of the subject, preferably extracellular vesicles of the subject, and detect the presence and content of central nervous system disease-associated nucleic acids by a DNA or RNA probe or sequencing technique.
45. A composition for detecting a central nervous system disease, comprising:
   an antibody, used for targeting a central nervous system-derived marker, and
   an antibody, used for targeting a central nervous system disease-associated marker.
46. The composition according to item 45, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.
47. The composition according to item 46, wherein
   the cerebrovascular diseases include cerebral infarction, cerebral hemorrhage, cerebral blood supply insufficiency, and transient ischemic attack;
   the spinal cord lesions include acute myelitis, polio, and syringomyelia;
   the central nervous system infections include encephalitis and meningitis;
   the inherited diseases of the nervous system include neurocutaneous syndrome and spinocerebellar ataxia;
   the dysplastic diseases of the nervous system include congenital hydrocephalus and cerebral palsy;
   the autonomic nervous system disease is autonomic nervous dysfunction; and
   the degenerative diseases of the nervous system include degenerative dementia, dyskinetic diseases, and prion diseases.
48. The composition according to item 47, wherein
   the degenerative dementia includes Alzheimer's disease (AD), frontotemporal dementia (FTD), Parkinson's disease dementia (PDD), and dementia with Lewy bodies (DLB); and
   the dyskinetic diseases include motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).
49. The composition according to any one of items 45 to 48, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.
50. The composition according to any one of items 45 to 49, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE),
   preferably, the central nervous system-derived markers are markers located on the surface of central nervous system-derived extracellular vesicles, and
   further preferably, the central nervous system-derived markers are selected from one or more of L1CAM, SHISA9, CNPase, and GLT1 (EAAT2).
51. The composition according to any one of items 45 to 50, wherein the central nervous system disease-associated markers are selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S 129 (pS129), oligomeric α-synuclein (oligo-α-syn), and prion protein.
52. The composition according to any one of items 45 to 51, wherein the antibody used for targeting central nervous system-derived markers and the antibody used for targeting central nervous system disease-associated markers are labeled antibodies, and preferably, the labeling is selected from one or more of fluorescence labeling, isotope labeling, enzyme labeling, chemiluminescence labeling, quantum dot labeling, and colloidal gold labeling.
53. A kit for detecting a central nervous system disease in a subject, comprising:
   a reagent, used for detecting a central nervous system-derived marker in a biological fluid of a subject, and
   a reagent, used for detecting a central nervous system disease-associated marker in the biological fluid of the subject.
54. The kit according to item 53, wherein the reagent used for detecting a central nervous system-derived marker in a biological fluid of a subject and the reagent used for detecting a central nervous system disease-associated marker in the biological fluid of the subject are the composition according to any one of items 45 to 52.
55. The kit according to item 53, wherein
   the reagent used for detecting a central nervous system-derived marker in a biological fluid of a subject is a primer or a probe targeting the central nervous system-derived marker, and
   the reagent used for detecting a central nervous system disease-associated marker in the biological fluid of the subject is a primer or a probe targeting the central nervous system disease-associated marker.
56. The kit according to item 53, further comprising:
   a reagent and an apparatus, used for acquiring the biological sample of the subject, and preferably, a reagent and an apparatus, used for acquiring extracellular vesicles of the subject.
57. The kit according to item 53, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.
58. The kit according to item 57, wherein
   the cerebrovascular diseases include cerebral infarction, cerebral hemorrhage, cerebral blood supply insufficiency, and transient ischemic attack;
   the spinal cord lesions include acute myelitis, polio, and syringomyelia;
   the central nervous system infections include encephalitis and meningitis;
   the inherited diseases of the nervous system include neurocutaneous syndrome and spinocerebellar ataxia;
   the dysplastic diseases of the nervous system include congenital hydrocephalus and cerebral palsy;
   the autonomic nervous system disease is autonomic nervous dysfunction; and
   the degenerative diseases of the nervous system include degenerative dementia, dyskinetic diseases, and prion diseases.
59. The kit according to item 58, wherein
   the degenerative dementia includes Alzheimer's disease (AD), frontotemporal dementia (FTD), Parkinson's disease dementia (PDD), and dementia with Lewy bodies (DLB); and
   the dyskinetic diseases include motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).

### EFFECT OF THE INVENTION

Using the method, the system, the kit, and the composition of this application, it is possible to diagnose whether a subject suffers from a central nervous system disease using a peripheral body fluid of the subject only.

Using the method, the system, the kit, and the composition of this application, biomarkers capable of reflecting changes in a central nervous system disease in a peripheral body fluid can be effectively enriched and specifically labeled, which provides a better diagnosis effect compared to direct detection of disease-associated markers in a peripheral body fluid.

Using the method, the system, the kit, and the composition of this application, a peripheral body fluid can be effectively used for detection, so that invasive acquisition of cerebrospinal fluid of the subject can be avoided, the risk of sampling the subject is greatly reduced, the difficulty of detection is greatly reduced, and the clinical applicability and generalization of detection are greatly increased.

Using the method, the system, the kit, and the composition of this application, central nervous system-derived markers and disease-associated markers can be detected simultaneously to reduce the sample detection time, so this application is more efficient and convenient.

The core of this application is a detection method and system using dual markers and/or multiple markers, which can realize the simultaneous detection of dual markers and/or multiple markers, this is, markers are used for determining biological samples, especially central nervous system-derived extracellular vesicles, and on this basis, disease-associated markers are used, and generated common detection results are used for diagnosing, differentially diagnosing, and tracking a disease and assessing a drug.

### BRIEF DESCRIPTION OF THE DRAWINGS

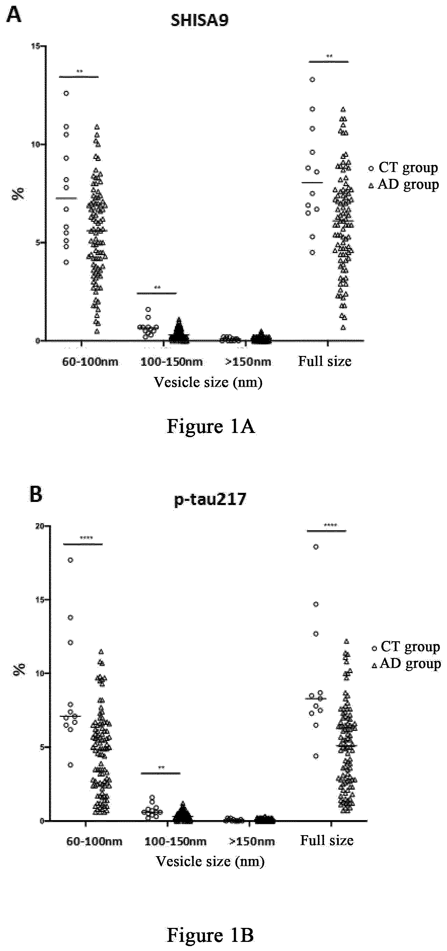

Fig. 1A shows that in Example 1, there is a significant difference (** indicates p<0.01) between an AD group and a healthy control group (CT group) in terms of the ratio of the number of single-labeled SHISA9 protein-positive extracellular vesicles to the total numbers of extracellular vesicles in plasma with a particle size of 60-100 nm, 100-150 nm and the full particle size range;
Fig. 1B shows that in Example 1, there is an extremely significant difference (**** indicates p<0.0001) between the AD group and the CT group in terms of the ratio of the number of single-labeled p-tau217 protein-positive extracellular vesicles to the total number of extracellular vesicles in plasma with a particle size of 60-100 nm and the full particle size range, and there is a significant difference (** indicates p<0.01) between the AD group and the CT group in terms of the ratio of the number of single-labeled p-tau217 protein-positive extracellular vesicles to the total number of extracellular vesicles in plasma with a particle size of 100-150 nm;

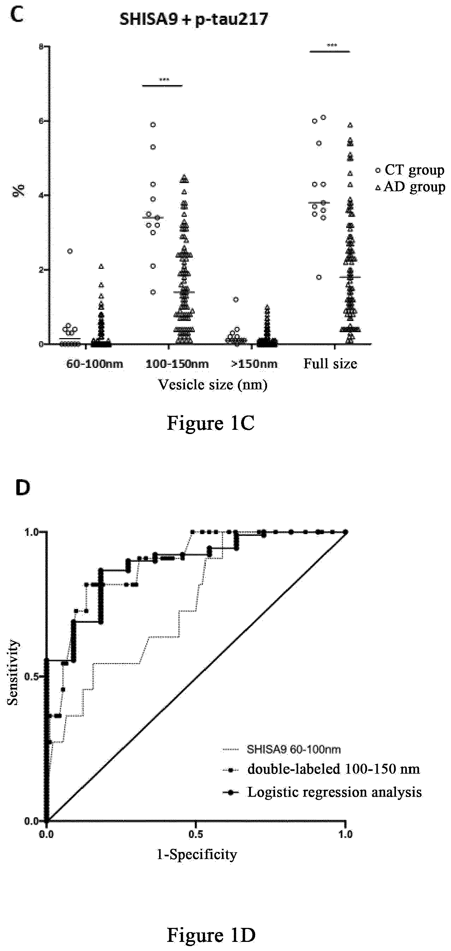

Fig. 1C shows that in Example 1, there is an extremely significant difference (*** indicates p<0.001) between the AD group and the CT group in terms of the ratio of the number of SHISA9 protein- and p-tau217 protein-positive extracellular vesicles to the total number of extracellular vesicles in plasma with a particle size of 100-150 nm and the full particle size range;
Fig. 1D shows results of receiver operating characteristic curve (ROC) analysis in Example 1: the area under curve (AUC) of single-labeled SHISA9 protein-positive extracellular vesicles with a particle size of 60-100 nm is 0.749, the AUC of double-labeled SHISA9 protein- and p-tau217 protein-positive extracellular vesicles with a particle size of 100-150 nm is 0.890, and the data of SHISA9 60-100 nm and SHISA9+p-tau217 100-150 nm is entered by logistic regression analysis Enter to obtain an AUC of 0.891; and

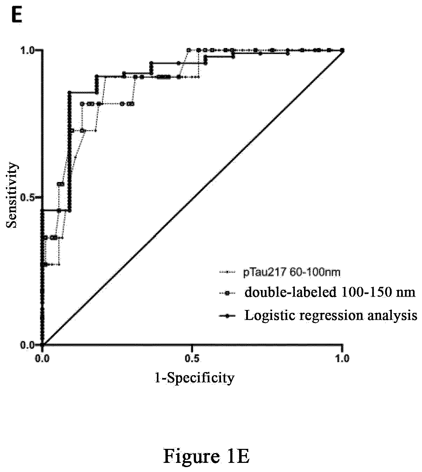

Fig. 1E shows results of ROC analysis in Example 1: the AUC of single-labeled p-tau217 protein-positive extracellular vesicles with a particle size of 60-100 nm is 0.877, the AUC of double-labeled SHISA9 protein- and p-tau217 protein-positive extracellular vesicles with a particle size of 100-150 nm is 0.890, and the data of p-tau217 60-100 nm and SHISA9+p-tau217 100-150 nm is entered by logistic regression analysis Enter to obtain an AUC of 0.910.

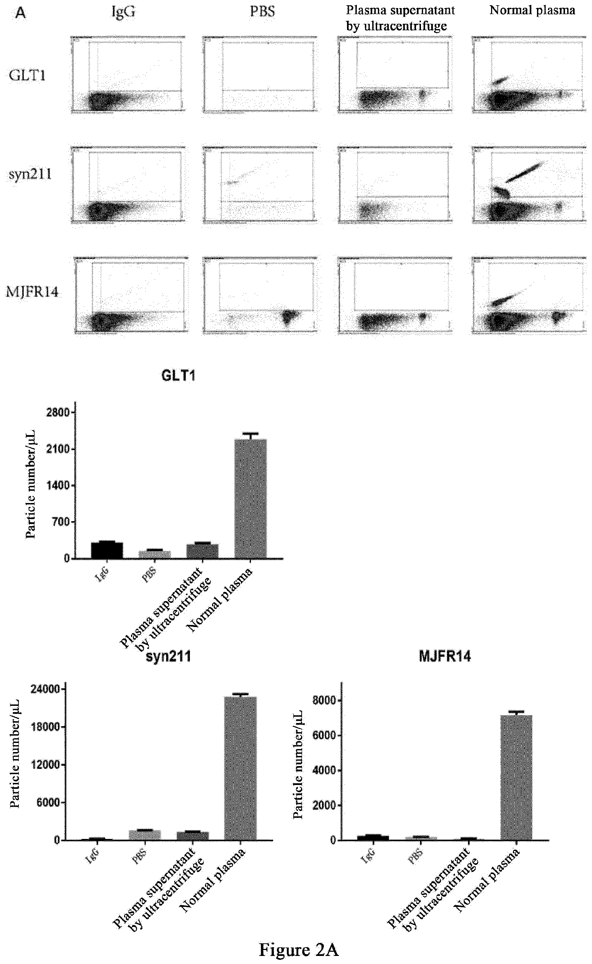

Fig. 2A shows the number of single-labeled GLT1 protein-positive extracellular vesicles and the number of single-labeled α-syn protein (syn211 and MJFR14 are anti-α-syn protein antibodies)-positive extracellular vesicles in plasma in Example 2; and

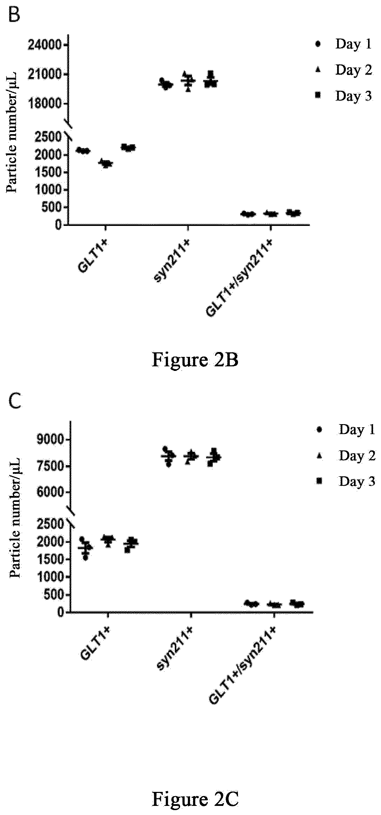

Figs. 2B and Fig. 2C show the number of single-labeled GLT1 protein- or α-syn protein-positive extracellular vesicles and the number of GLT1 protein- and α-syn protein-dual positive extracellular vesicles in plasma in Example 2.

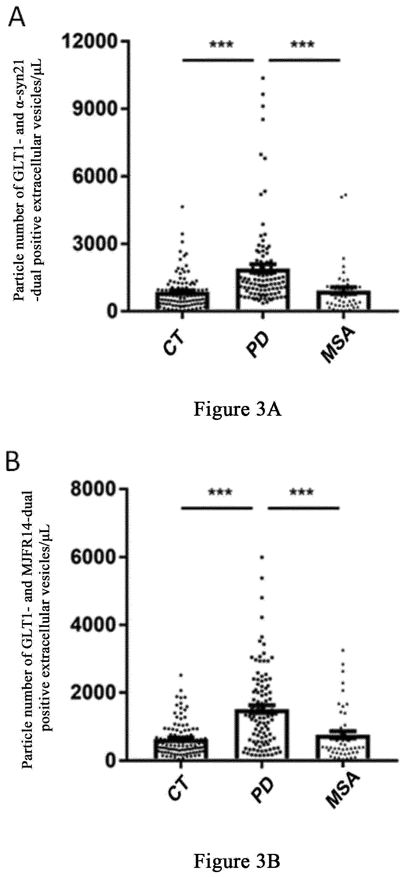

Fig. 3A and Fig. 3B show that in Example 2, in terms of the number of GLT1 protein- and α-syn protein-dual positive extracellular vesicles in plasma, a PD group is extremely significantly (*** indicates p<0.001) greater than an MSA group or a CT group, while there is no significant difference between the MSA group and the CT group.

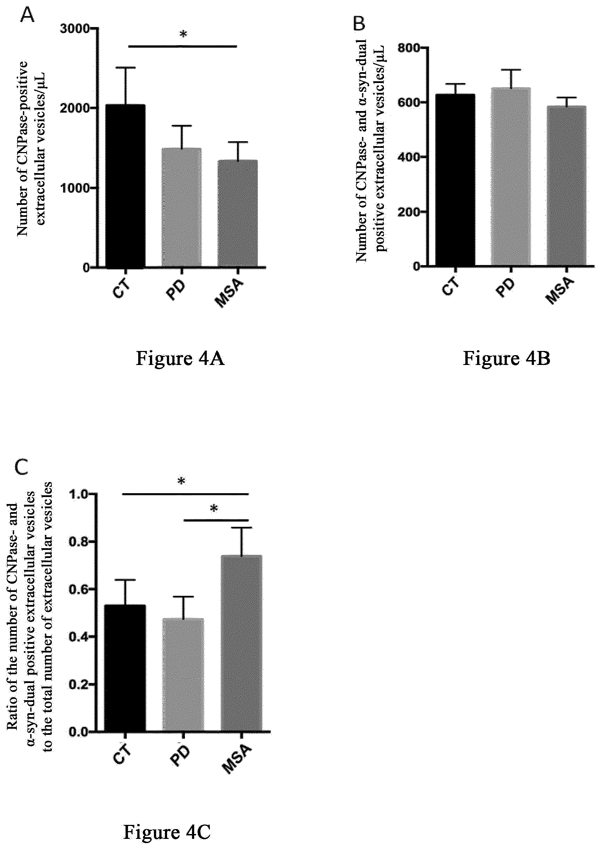

Fig. 4A shows that in Example 3, in terms of the number of CNPase protein-positive extracellular vesicles in plasma, there is no significant difference between an MSA group and a PD group, while there is a significant difference (* indicates p<0.05) between the MSA group and an HC group;
Fig. 4B shows that in Example 3, in terms of the number of CNPase protein- and α-syn protein (labeled with an anti-MJFR14 antibody)-dual positive extracellular vesicles in plasma, there is no significant difference between the MSA group and the PD group, and the MSA group and the HC group; and
Fig. 4C shows that in Example 3, in terms of the number of CNPase protein- and α-syn protein-dual positive extracellular vesicles in plasma, after it is normalized by the total number of extracellular vesicles in plasma, there is a significant difference (* indicates p<0.05) between the MSA group and the HC group, and the MSA group and the PD group.

### DETAILED DESCRIPTION OF THE INVENTION

Specific embodiments of the present application will be described in more detail below with reference to the accompanying drawings. While specific embodiments of the present application have been illustrated in the accompanying drawings, it is to be understood that the present application may be embodied in various forms and should not be construed as limited to the embodiments set forth herein. Rather, these embodiments are provided so that the present application will be thorough and complete, and will fully convey the scope of the present application to those skilled in the art.

It is noted that certain terms are used throughout the description and claims to refer to particular components. Those skilled in the art will appreciate that a skilled artisan may refer to the same component by different terms. The description and claims do not use differences in terms to distinguish one component from another, but rather use functional differences to distinguish one component from another. As used throughout the description and claims, the terms "include" and "comprise" are to be construed in an open-ended fashion, and thus should be interpreted to mean "comprising, but not limited to". While the following description sets forth preferred embodiments for carrying out the present application, the description sets forth general principles of the present application and is not intended to limit the scope of the present application. The scope of protection of the present application should be determined with reference to the appended claims.

In a specific embodiment of this application, this application relates to a method for diagnosing a central nervous system disease, comprisingcomprising: detecting a central nervous system-derived marker in a biological fluid of a subject, detecting a central nervous system disease-associated marker in the biological fluid of the subject, and diagnosing whether the subject suffers from a central nervous system disease based on detection results of the central nervous system-derived marker and the central nervous system disease-associated marker.

In a specific embodiment of this application, this application relates to a method for diagnosing a central nervous system disease, comprising: detecting a central nervous system-derived marker in a biological fluid of a subject, detecting a central nervous system disease-associated marker in the biological fluid of the subject, and diagnosing whether the subject suffers from a central nervous system disease based on detection results of the central nervous system-derived marker and the central nervous system disease-associated markercomprising, wherein the detecting a central nervous system-derived marker in a biological fluid of a subject and the detecting a central nervous system disease-associated marker in the biological fluid of the subject are performed simultaneously.

In a specific embodiment of this application, this application relates to a system for diagnosing a central nervous system disease, comprising: a first detection module, configured to detect a central nervous system-derived marker in a biological fluid of a subject, a second detection module, configured to detect a central nervous system disease-associated marker in the biological fluid of the subject, and a diagnosis module, configured to diagnose whether the subject suffers from a central nervous system disease based on detection results of the central nervous system-derived marker and the central nervous system disease-associated marker respectively obtained by the first detection module and the second detection modulecomprising.

In a specific embodiment of this application, this application relates to a system for diagnosing a central nervous system disease, comprising: a first detection module, configured to detect a central nervous system-derived marker in a biological fluid of a subject, a second detection module, configured to detect a central nervous system disease-associated marker in the biological fluid of the subject, and a diagnosis module, configured to diagnose whether the subject suffers from a central nervous system disease based on detection results of the central nervous system-derived marker and the central nervous system disease-associated marker respectively obtained by the first detection module and the second detection modulecomprising. The first detection module and the second detection module simultaneously detect the central nervous system-derived marker and the central nervous system disease-associated marker in the biological fluid of the subject; or the first detection module and the second detection module are the same module configured to simultaneously detect the central nervous system-derived marker and the central nervous system disease-associated marker in the biological fluid of the subject.

As described above, in the method and system of this application, the inventors have inventively developed a novel method and system, that is, detect using dual markers and/or multiple markers that have different effects, and particularly, simultaneously detect using such dual markers and/or multiple markers, so as to effectively detect a central nervous system disease using a peripheral body fluid only. The detection method and system does not require extraction extraction of cerebrospinal fluid from a patient, thereby greatly reducing the risk of sampling the patient. Meanwhile, due to the use of dual labeling and/or multiple labeling with markers capable of identifying the source of the central system and markers associated with the disease to be detected, the accuracy of detection can be greatly improved, and the overall efficiency of detection can be improved if the detection is further performed simultaneously. However, other existing methods often require a more complex extraction, enrichment, or purification step to obtain a biological sample for diagnosis. This application simplifies the operation steps, inventively uses central nervous system-derived markers to label the source, and combines with disease-associated markers, so as to improve the accuracy and efficiency of disease detection.

Further, the method of this application further comprising: acquiring the biological fluid of the subject, and pretreating the biological fluid to enrich biological samples in the biological fluid, the detecting a central nervous system-derived marker in a biological fluid of a subject referring to detecting a central nervous system-derived marker in enriched biological samples of the subject, and the detecting a central nervous system disease-associated marker in the biological fluid of the subject referring to detecting a central nervous system disease-associated marker in the enriched biological samples of the subject.

Further, the method of this application further comprising: counting biological samples in which both of the central nervous system-derived marker and the central nervous system disease-associated marker are detected, and/or measuring particle sizes of the biological samples in which both of the central nervous system-derived marker and the central nervous system disease-associated marker are detected, and diagnosing whether the subject suffers from a central nervous system disease based on the number of the biological samples and/or the particle sizes of the biological samples. Specifically, a biological sample in which both of a central nervous system-derived marker and a central nervous system disease-associated marker are detected is generally referred to as a dual positive biological sample, and whether the subject suffers from a central nervous system disease is diagnosed based on the number and/or particle sizes of dual positive biological samples.

Further, the system of this application further comprising: an enrichment module configured to acquire the biological fluid of the subject and pretreat the biological fluid to enrich biological samples in the biological fluid, wherein the first detection module and the second detection module detect the central nervous system-derived marker and the central nervous system disease-associated marker in enriched biological samples of the subject.

Further, the system of this application further comprising: a counting module configured to count biological samples in which both of the central nervous system-derived marker and the central nervous system disease-associated marker are detected, and/or a particle size measurement module configured to measure particle sizes of the biological samples in which both of the central nervous system-derived marker and the central nervous system disease-associated marker are detected. The diagnosis module diagnoses whether the subject suffers from a central nervous system disease based on the number of the biological samples and/or the particle sizes of the biological samples. Specifically, a biological sample in which both of a central nervous system-derived marker and a central nervous system disease-associated marker are detected is generally referred to as a dual positive biological sample, and whether the subject suffers from a central nervous system disease is diagnosed based on the number and/or particle sizes of dual positive biological samples.

This application also relates to a composition for detecting a central nervous system disease, comprising: an antibody used for targeting central nervous system-derived markers, and an antibody used for targeting central nervous system disease-associated markers.

This application also relates to a kit for detecting a central nervous system disease in a subject, comprising: a reagent used for detecting a central nervous system-derived marker in a biological fluid of a subject, and a reagent used for detecting a central nervous system disease-associated maker in the biological fluid of the subject.

In a specific embodiment, the reagent used for detecting a central nervous system-derived marker in a biological fluid of a subject and the reagent for detecting a central nervous system disease-associated marker in the biological fluid of the subject are the above composition of this application.

In a specific embodiment, the reagent used for detecting a central nervous system-derived marker in a biological fluid of a subject is a primer or a probe targeting central nervous system-derived markers, and the reagent used for detecting a central nervous system disease-associated marker in the biological fluid of the subject is a primer or a probe targeting central nervous system disease-associated markers.

In specific embodiments of the method or the system or the composition or the kit of this application, the biological fluid is blood, serum, plasma, saliva, urine, lymph, semen or milk. A preferable biological fluid is blood, serum, plasma, saliva or urine. The method for acquiring the biological fluid of the subject may be any method known to those skilled in the art. Those skilled in the art can select a suitable method according to a biological fluid to be acquired from a subject.

In a specific embodiment, the biological samples of this application are extracellular vesicles. As referred to herein, extracellular vesicles (EVs) refer to vesicular bodies that have a doublemembrane structure shed from the cell membrane or secreted by cells, with a diameter of 40-1000 nm. Extracellular vesicles mainly composed of microvesicles (MVs) and exosomes (Exs), and microvesicles are small vesicles that shed from the cell membrane after cell activation, damage or apoptosis, with a diameter of about 100-1000 nm. Exosomes are released outside cells in the form of exosomes after fusion of intracellular multivesicular bodies with the cell membrane, with a diameter of about 30-150 nm, can be released by many different types of cells, and exert different cell functions, comprising cell-cell communication, antigen presentation, and protein and nucleic acid transfer. Extracellular vesicles are widely present in cell culture supernatants and a variety of body fluids (blood, lymph, saliva, urine, semen, and milk), carry a variety of cell-derived proteins, lipids, DNA, mRNA, miRNA, etc., and are involved in processes such as cell-cell communication, cell migration, angiogenesis, and immune regulation. In a specific embodiment, preferably, the extracellular vesicles are neuron-derived extracellular vesicles, astrocyte-derived extracellular vesicles, oligodendrocyte-derived extracellular vesicles or microglia-derived extracellular vesicles.

In specific embodiments of the method or the system or the composition or the kit of this application, the method for acquiring the biological fluid of the subject and pretreating the biological fluid to enrich biological samples, especially extracellular vesicles, in the biological fluid may be centrifugation, ultracentrifugation, ultrafiltration tube filtration, polymeric sedimentation or specific antibody capture. Herein, centrifugation, ultracentrifugation, ultrafiltration tube filtration, polymeric sedimentation, and specific antibody capture all have the meaning understood by those skilled in the art, and those skilled in the art can select a suitable method according to extracellular vesicles to be acquired.

In an embodiment of the method or the system of this application, ultracentrifugation is used to enrich extracellular vesicles in a biological fluid, and particularly, enrich neuron-derived extracellular vesicles, astrocyte-derived extracellular vesicles, oligodendrocyte-derived extracellular vesicles or microglia-derived extracellular vesicles.

In an embodiment of the method or the system of this application, ordinary centrifugation or ultrafiltration tube filtration is used to enrich extracellular vesicles, and particularly, enrich neuron-derived extracellular vesicles, astrocyte-derived extracellular vesicles, oligodendrocyte-derived extracellular vesicles or microglia-derived extracellular vesicles.

In specific embodiments of the method or the system or the composition or the kit of this application, the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.

In specific embodiments of the method or the system or the composition or the kit of this application, the cerebrovascular diseases include cerebral infarction, cerebral hemorrhage, cerebral blood supply insufficiency, and transient ischemic attack; the spinal cord lesions include acute myelitis, polio, and syringomyelia; the central nervous system infections include encephalitis and meningitis; the inherited diseases of the nervous system include neurocutaneous syndrome and spinocerebellar ataxia; the dysplastic diseases of the nervous system include congenital hydrocephalus and cerebral palsy; the autonomic nervous system disease is autonomic nervous dysfunction, and the degenerative diseases of the nervous system include degenerative dementia, dyskinetic diseases, and prion diseases. Further, the degenerative dementia includes Alzheimer's disease (AD), frontotemporal dementia (FTD), Parkinson's disease dementia (PDD), and dementia with Lewy bodies (DLB); and the dyskinetic diseases include motor neuron diseases (MNDs), Parkinson's disease (PD), Huntington's disease (HD), Sydenham's chorea, Wilson's disease, multiple system atrophy (MSA), progressive supranuclear palsy (PSP), and corticobasal degeneration (CBD).

In specific embodiments of the method or the system or the composition or the kit of this application, the central nervous system disease is Alzheimer's disease.

In specific embodiments of the method or the system or the composition or the kit of this application, the central nervous system disease is Parkinson's disease.

In specific embodiments of the method or the system or the composition or the kit of this application, the central nervous system-derived markers and/or the central nervous system disease-associated markers are each selected from one or two or three of proteins, nucleic acids, and lipids.

In specific embodiments of the method or the system or the composition or the kit of this application, the central nervous system-derived markers and the central nervous system disease-associated markers are proteins.

In specific embodiments of the method or the system or the composition or the kit of this application, the central nervous system-derived markers and the central nervous system disease-associated markers are nucleic acids.

In specific embodiments of the method or the system or the composition or the kit of this application, the central nervous system-derived markers and the central nervous system disease-associated markers are lipids.

In specific embodiments of the method or the system or the composition or the kit of this application, the central nervous system-derived markers are proteins, and the central nervous system disease-associated markers are nucleic acids.

In specific embodiments of the method or the system or the composition or the kit of this application, the central nervous system-derived markers are nucleic acids, and the central nervous system disease-associated markers are proteins.

In specific embodiments of the method or the system or the composition or the kit of this application, the central nervous system-derived markers are proteins, and the central nervous system disease-associated markers are lipids.

In specific embodiments of the method or the system or the composition or the kit of this application, the central nervous system-derived markers are nucleic acids, and the central nervous system disease-associated markers are lipids.

In specific embodiments of the method or the system or the composition or the kit of this application, the central nervous system-derived markers are lipids, and the central nervous system disease-associated markers are proteins.

In specific embodiments of the method or the system or the composition or the kit of this application, the central nervous system-derived markers are lipids, and the central nervous system disease-associated markers are nucleic acids.

In specific embodiments of the method or the system or the composition or the kit of this application, the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons. In specific embodiments of the method or the system or the composition or the kit of this application, preferably, the central nervous system-derived markers are neuron-derived biomarkers. In specific embodiments of the method or the system or the composition or the kit of this application, preferably, the central nervous system-derived markers are astrocyte- and/or oligodendrocyte-derived biomarkers.

In specific embodiments of the method or the system or the composition or the kit of this application, the central nervous system-derived markers are markers located on the surface of central nervous system-derived extracellular vesicles.

In specific embodiments of the method or the system or the composition or the kit of this application, the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE).

In specific embodiments of the method or the system or the composition or the kit of this application, the central nervous system-derived markers are selected from one or more of L1CAM, SHISA9, CNPase, and GLT1 (EAAT2). Among these markers, L1CAM and SHISA9 are neuron-derived biomarkers, GLT1 (EAAT2) is an astrocyte-derived biomarker, and CNPase is an oligodendrocyte-derived biomarker.

In specific embodiments of the method or the system or the composition or the kit of this application, mature neuron-derived biomarkers include L1CAM, SHISA9, contactin-1 (CNTN1), NeuN, MAP2, neurofilament light (NF-L), neurofilament medium (NF-M), neurofilament heavy (NF-H), synaptophysin, syntaxin, PSD95, etc.

In specific embodiments of the method or the system or the composition or the kit of this application, oligodendrocyte-derived biomarkers include CNPase, olig 1, olig 2, olig 3, MBP, OSP, MOG, SOX10, NG2, etc. Herein, CNPase refers to a 2',3'-cyclic nucleotide 3'-phosphodiesterase.

In specific embodiments of the method or the system or the composition or the kit of this application, astrocyte-derived biomarkers include GFAP, GLAST (EAAT1), GLT1 (EAAT2), glutamine synthetase, S100-β, ALDH1L1, etc. Herein, GLT1 (EAAT2) refers to glutamate transporter 1. GLT1 (EAAT2) is an astrocyte-specific glutamate transporter in the central nervous system and is an astrocyte-derived marker.

In specific embodiments of the method or the system or the composition or the kit of this application, microglia-derived biomarkers include CD11b, CD45, Iba1, F4/80, CD68, CD40, etc.

In specific embodiments of the method or the system or the composition or the kit of this application, immature neuron-derived biomarkers include doublecortin, beta III tubulin, NeuroD1, TBR1, stathmin 1, etc.

In specific embodiments of the method or the system or the composition or the kit of this application, Schwann cell-derived biomarkers include MPZ, NCAM, GAP43, S100, etc.

In specific embodiments of the method or the system or the composition or the kit of this application, radial glia-derived biomarkers include vimentin, nestin, PAX6, HES1, HES5, GFAP, GLAST, BLBP, TN-C, N-cadherin, SOX2, etc.

In specific embodiments of the method or the system or the composition or the kit of this application, intermediate progenitor-derived biomarkers include TBR2, MASH1 (Ascl1), etc.

In specific embodiments of the method or the system or the composition or the kit of this application, glutamatergic neuron-derived biomarkers include vGluT1, vGluT2, NMDAR, glutaminase, glutamine synthetase, etc.

In specific embodiments of the method or the system or the composition or the kit of this application, GABAergic neuron-derived biomarkers include GABA transporter 1 (GAT1), GABA_{B} receptor 1, GABA_{B} receptor 2, GAD65, GAD67, etc.

In specific embodiments of the method or the system or the composition or the kit of this application, dopaminergic neuron-derived biomarkers include tyrosine hydroxylase (TH), dopamine transporter (DAT), FOXA2, GIRK2, Nurr1, LMX1B, etc.

In specific embodiments of the method or the system or the composition or the kit of this application, serotonergic neuron-derived biomarkers include tryptophan hydroxylase (TPH), serotonin transporter, Petl, etc.

In specific embodiments of the method or the system or the composition or the kit of this application, cholinergic neuron-derived biomarkers include choline acetyl transferase (ChAT), vesicular acetylcholine transporter (VAChT), acetylcholinesterase (AchE), etc.

In specific embodiments of the method or the system or the composition or the kit of this application, the central nervous system disease-associated markers are proteins selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.

In specific embodiments of the method or the system or the composition or the kit of this application, biomarkers associated with Alzheimer's disease (AD) include, but are not limited to, Aβ1-40 (Aβ40) protein, Aβ1-42 (Aβ42) protein, oligomeric Aβ (oligo-Aβ) protein, tau protein, and phosphorylated tau (p-tau) protein.

In specific embodiments of the method or the system or the composition or the kit of this application, biomarkers associated with Parkinson's disease (PD) and Parkinson's syndromes, comprising multiple system atrophy (MSA), progressive supranuclear palsy (PSP), corticobasal degeneration (CBD), Parkinson's disease dementia (PDD), and dementia with Lewy bodies (DLB), include, but are not limited to, α-synuclein, phosphorylated α-synuclein at position S 129 (pS129), oligomeric α-synuclein (oligo-α-syn), tau protein, and phosphorylated tau (p-tau) protein. Among them, α-synuclein (α-syn protein) is the main protein component of a Lewy body, and numerous studies show that it is closely associated to the pathogenesis of Parkinson's disease.

In specific embodiments of the method or the system or the composition or the kit of this application, a prion disease-associated biomarker is prion protein.

In specific embodiments of the method or the system or the composition or the kit of this application, the central nervous system disease-associated markers are nucleic acids selected from one or more of DNA or RNA serving as biomarkers associated with Alzheimer's disease (AD), and DNA or RNA serving as biomarkers associated with Parkinson's disease (PD) and Parkinson's syndromes (e.g., multiple system atrophy (MSA), progressive supranuclear palsy (PSP), corticobasal degeneration (CBD), and dementia with Lewy bodies (DLB)).

In specific embodiments of the method or the system or the composition or the kit of this application, the DNA or RNA serving as biomarkers associated with AD includes DNA or RNA associated with genes UBAP2L, YBX1, SERF2, UBE2B, RPL10A, H3F3AP4, PPP2CA, NMD3, RNF7, RPLPO, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, or GREB1. The reagent used for detecting a central nervous system-derived marker in a biological fluid of a subject is a primer or a probe targeting central nervous system-derived markers, and particularly, is a primer or a probe targeting the above DNA or RNA.

In specific embodiments of the method or the system or the composition or the kit of this application, the DNA or RNA serving as biomarkers associated with PD includes DNA or RNA associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC 100132832, or BLOC 1S 1-RDH5. The reagent used for detecting a central nervous system-derived marker in a biological fluid of a subject is a primer or a probe targeting central nervous system-derived markers, and particularly, is a primer or a probe targeting the above DNA or RNA.

In specific embodiments of the method or the system or the composition or the kit of this application, the central nervous system-derived markers are proteins, and the step of detecting a central nervous system-derived marker in a biological fluid of a subject includes: reacting the biological fluid of the subject, preferably extracellular vesicles of the subject, with a labeled antibody that specifically reacts with central nervous system-derived markers, and detecting the intensity of a labeled signal after the reaction to determine the presence and content of the central nervous system-derived markers; the central nervous system disease-associated markers are proteins, and the step of detecting a central nervous system disease-associated marker in the biological fluid of the subject includes: reacting the biological fluid of the subject, preferably extracellular vesicles of the subject, with a labeled antibody that specifically reacts with central nervous system disease-associated markers, and detecting the intensity of a labeled signal after the reaction to determine the presence and content of the central nervous system disease-associated markers. Further, in specific embodiments, preferably, the extracellular vesicles of the subject simultaneously react with the labeled antibody that specifically reacts with central nervous system-derived markers and the labeled antibody that specifically reacts with central nervous system disease-associated markers, thereby efficiently tracking dual-marker and/or multi-marker positive extracellular vesicles.

Further, in specific embodiments of the method or the system or the composition or the kit of this application, further comprising: counting biological samples (e.g., extracellular vesicles) in which both of a central nervous system-derived marker and a central nervous system disease-associated marker are detected, and/or measuring particle sizes of the biological samples (e.g., extracellular vesicles) in which both of a central nervous system-derived marker and a central nervous system disease-associated marker are detected, such that after dual-marker and/or multi-marker positive biological samples, such as extracellular vesicles, are tracked, the number and/or particle sizes of such biological samples may also be sufficiently taken into account, results of these indicators are combined to analyze the risk of a subject suffering from a disease.

In specific embodiments of the method or the system or the composition or the kit of this application, the labeling is selected from one or more of fluorescence labeling, isotope labeling, enzyme labeling, chemiluminescence labeling, quantum dot labeling, and colloidal gold labeling.

In specific embodiments of the method or the system or the composition or the kit of this application, a fluorescence label is selected from one or more of Qdot 525, Qdot 545, Qdot 565, Qdot 585, Qdot 605, Qdot 625, Qdot 655, Qdot 705, Qdot 800, Alexa 350, Alexa 405, Alexa 488, Alexa 532, Alexa 546, Alexa 555, Alexa 568, Alexa 594, Alexa 647, Alexa 700, and Alexa 750.

In specific embodiments of the method or the system or the composition or the kit of this application, isotope labeling, enzyme labeling, chemiluminescence labeling, quantum dot labeling, or colloidal gold labeling can be performed by methods well known to those skilled in the art.

In specific embodiments of the method or the system or the composition or the kit of this application, the central nervous system-derived markers are nucleic acids, and the step of detecting a central nervous system-derived marker in a biological fluid of a subject includes: lysing the biological fluid of the subject, preferably extracellular vesicles of the subject, and detecting the presence and content of central nervous system-derived nucleic acids by a DNA or RNA probe or sequencing technique; the central nervous system disease-associated markers are nucleic acids, and the step of detecting a central nervous system disease-associated marker in the biological fluid of the subject includes: lysing the biological fluid of the subject, preferably extracellular vesicles of the subject, and detecting the presence and content of central nervous system disease-associated nucleic acids by a DNA or RNA probe or sequencing technique. Further, in specific embodiments, preferably, the presence and content of central nervous system-derived nucleic acids and the presence and content of central nervous system disease-associated nucleic acids in the extracellular vesicles of the subject are simultaneously detected by DNA or RNA probes, thereby efficiently tracking dual-marker and/or multi-marker positive extracellular vesicles.

In specific embodiments of the method or the system or the composition or the kit of this application, the detection of the intensity of a labeled signal after the reaction to determine the presence and content of central nervous system-derived markers can be performed by, for example, immunoassay such as ELISA, Luminex, MSD, Quanterix, Singulex, eCL8000, and Cobas that are preferred methods for measuring protein biomarkers.

In specific embodiments of the method or the system or the composition or the kit of this application, detection instruments for counting biological samples in which both of a central nervous system-derived marker and a central nervous system disease-associated marker are detected, and/or measuring particle sizes of the biological samples in which both of a central nervous system-derived marker and a central nervous system disease-associated marker are detected include, but are not limited to, a particle size analyzer and a nanoparticle flow cytometer. For example, a particle size of an extracellular vesicle is measured by a nanoparticle tracking technique, and the above dual positive extracellular vesicles are counted by a nanoparticle flow analysis technique.

In specific embodiments of the method or the system or the composition or the kit of this application, in a case that nucleic acids serve as biomarkers, it is necessary to lyse a biological fluid or extracellular vesicles before nucleic acid biomarkers are measured. Nucleic acids may be detected by any method known to those skilled in the art, such as a DNA or RNA probe, and any known sequencing technique.

In a specific embodiment, this application relates to a composition for detecting a central nervous system disease, comprising: an antibody used for targeting central nervous system-derived markers, and an antibody used for targeting central nervous system disease-associated markers.

In a specific embodiment of this application, the antibody used for targeting central nervous system-derived markers and the antibody used for targeting central nervous system disease-associated markers are labeled antibodies, and preferably, the labeling is selected from one or more of fluorescence labeling, isotope labeling, enzyme labeling, chemiluminescence labeling, quantum dot labeling, and colloidal gold labeling.

In a specific embodiment, this application relates to a composition for detecting a central nervous system disease, comprising: an antibody used for targeting the SHISA9 protein, and an antibody used for targeting the phosphorylated tau217 (p-tau217) protein.

In a specific embodiment, this application relates to a composition for detecting a central nervous system disease, comprising: an antibody used for targeting the GLT1 (EAAT2) protein, and an antibody used for targeting the tau protein. In a specific embodiment, this application relates to a composition for detecting a central nervous system disease, comprising: an antibody used for targeting the GLT1 (EAAT2) protein, and an antibody for targeting the α-synuclein. In a specific embodiment, this application relates to a composition for detecting a central nervous system disease, comprising: an antibody used for targeting the GLT1 (EAAT2) protein, and an antibody used for targeting the oligomeric α-synuclein (oligo-α-syn). In a specific embodiment, this application relates to a composition for detecting a central nervous system disease, comprising: an antibody used for targeting the GLT1 (EAAT2) protein, and an antibody used for targeting the phosphorylated α-synuclein at position S129 (pS129). In a specific embodiment, this application relates to a composition for detecting a central nervous system disease, comprising: an antibody used for targeting the GLT1 (EAAT2) protein, and an antibody used for targeting the phosphorylated tau (p-tau) protein.

In a specific embodiment, this application relates to a composition for detecting a central nervous system disease, comprising: an antibody used for targeting CNPase, and an antibody used for targeting the tau protein. In a specific embodiment, this application relates to a composition for detecting a central nervous system disease, comprising: an antibody used for targeting CNPase, and an antibody used for targeting the α-synuclein.

This application relates to a kit for detecting a central nervous system disease in a subject, comprising: a reagent used for detecting a central nervous system-derived marker in a biological fluid of a subject, and a reagent used for detecting a central nervous system disease-associated marker in the biological fluid of the subject. The reagent used for detecting a central nervous system-derived marker in a biological fluid of a subject and the reagent used for detecting a central nervous system disease-associated marker in the biological fluid of the subject may be the above composition of this application.

This application relates to a kit for detecting a central nervous system disease in a subject, comprising: a reagent used for detecting a central nervous system-derived marker in a biological fluid of a subject, and a reagent used for detecting a central nervous system disease-associated marker in the biological fluid of the subject. The reagent used for detecting a central nervous system-derived marker in a biological fluid of a subject is a primer or a probe targeting central nervous system-derived markers, and the reagent used for detecting a central nervous system disease-associated marker in the biological fluid of the subject is a primer or a probe targeting central nervous system disease-associated markers.

The kit of this application further comprises a reagent and an apparatus used for acquiring the biological sample of the subject, and preferably, a reagent and an apparatus used for acquiring extracellular vesicles of the subject. The reagent and apparatus used for acquiring extracellular vesicles may include commonly used kits and apparatuses known to those skilled in the art.

Using the method, the system, the kit, and the composition of this application, whether a subject suffers from a central nervous system disease can be diagnosed using a peripheral body fluid of the subject only. For example, diagnosis and differential diagnosis of diseases, such as Alzheimer's disease, Parkinson's disease, and multiple system atrophy, can be effectively achieved, thereby effectively distinguishing from healthy controls or other diseases.

Using the method, the system, the kit, and the composition of this application, biomarkers capable of reflecting changes in a central nervous system disease in a peripheral body fluid can be effectively enriched and specifically labeled, which provides a better diagnosis effect compared to direct detection of disease-associated markers in a peripheral body fluid. For example, central nervous system-derived extracellular vesicle biomarkers, such as the L1CAM protein, the SHISA9 protein, the GLT1 protein, and the CNPase protein, can be effectively used in combination with biomarkers capable of reflecting changes in a central nervous system disease, and in a preferred embodiment, central nervous system-derived extracellular vesicle biomarkers, such as the L1CAM protein, the SHISA9 protein, the GLT1 protein, and the CNPase protein, and biomarkers capable of reflecting changes in a central nervous system disease, such as the Aβ1-40 protein, the Aβ1-42 protein, the oligomeric Aβ (oligo-Aβ) protein, the tau protein, the phosphorylated tau (p-tau) protein, and the α-synuclein (α-syn), can be simultaneously detected, such that diagnosis is made based on results of simultaneous detection. Diagnosis can also be made based on the number of dual-marker positive extracellular vesicles in which both markers are simultaneously detected and/or particle sizes of such extracellular vesicles. For example, auxiliary calculation of the ratio of dual-marker positive extracellular vesicles to single-marker positive extracellular vesicles or total extracellular vesicles can also be performed to further improve a diagnosis effect.

Using the method, the system, the kit, and the composition of this application, a peripheral body fluid can be effectively used for detection, so that invasive acquisition of cerebrospinal fluid from a subject can be avoided, the risk of sampling the subject is greatly reduced, the difficulty of detection is greatly reduced, and the clinical applicability and generalization of detection are greatly increased. Using the method, the system, the kit, and the composition of this application, central nervous system-derived markers and disease-associated markers can be detected simultaneously to reduce the sample detection time, and the detection is more efficient and convenient.

When the method, the system, the composition or the kit of this application is used, detection of the SHISA9 protein and the phosphorylated tau217 (p-tau217) protein that serve as dual markers can be effectively performed to distinguish patients with Alzheimer's disease from healthy populations.

When the method, the system, the composition or the kit of this application is used, detection of the GLT1 protein and the α-syn protein that serve as dual markers can be effectively performed to distinguish patients with Parkinson's disease from patients with multiple system atrophy and healthy populations.

When the method, the system, the composition or the kit of this application is used, detection of the CNPase protein and the α-syn protein that serve as dual markers can be effectively performed to distinguish patients with multiple system atrophy from patients with Parkinson's disease and healthy populations.

### EXAMPLES

Experimental method 1:
(a) Pretreatment of the peripheral body fluid: enrichment of extracellular vesicles by ultracentrifugation
   1. The plasma of a subjects is dissolved in an incubator at 37°C for 10-15 min and vortexmixed uniformly;
   2. The plasma is centrifuged at 4°C and 10,000 g for 30 min;
   3. 100 µL of the centrifuged plasma is sucked to an ultracentrifuge tube, and 900 µL of PBS (filtered with a filter of 0.22 µm) is added to and uniformly mixed with the plasma;
   4. The mixture is ultracentrifuged at 4°C and 100,000 g for 1 h;
   5. A supernatant is removed, and about 100 µL of precipitate is retained;
   6. 200 µL of PBS (filtered with a filter of 0.22 µm) is added, and the mixture is blown and sucked 200 times by using a pipettor with a measuring range of 200 µL for resuspension;
   7. 700 µL of PBS (filtered with a filter of 0.22 µm) is added to and uniformly mixed with the resuspended liquid;
   8. The mixture is ultracentrifuged at 4°C and 100,000 g for 1 h;
   9. A supernatant is removed, and about 50 µL of precipitate is retained;
   10. 100 µL of PBS (filtered with a filter of 0.22 µm) is added, and the mixture is blown and sucked 200 times by using a pipettor with a measuring range of 200 µL for resuspension and later use.
(b) Labeling of antibodies for detection of biomarkers by labeling methods

### Reagents used:

Alexa fluorescent labels are used to label an antibody targeting central nervous system-derived extracellular vesicle biomarkers and an antibody targeting Alzheimer's disease-associated biomarkers. The antibody targeting central nervous system-derived extracellular vesicle biomarkers is an SHISA9 polyclonal antibody. The antibody targeting Alzheimer's disease-associated biomarkers is a p-tau217 polyclonal antibody. Labeling reagents are a SHISA9 polyclonal antibody label purchased from Zenon^{™} Alexa Fluor^{™} 405 and a p-tau217 polyclonal antibody label purchased from Zenon^{™} Alexa Fluor^{™} 488.
1. Blocking of sample
   1.1 10 µL of extracellular vesicles ultracentrifuged and separated by the above method is transferred to a 1.5 mL centrifuge tube or PCR tube;
   1.2 An equal volume of 2% BSA solution (filtered with a filter of 0.22 µm) is added to and uniformly mixed with the extracellular vesicles;
   1.3 The mixture is incubated and blocked at the room temperature for 1 h;
   1.4 10 µL of PBS (filtered with a filter of 0.22 µm) is added to dilute the blocked sample.
2. Labeling of sample
   This experiment involves a nerve-specific marker antibody SHISA9 and a disease-associated marker antibody p-tau217; SHISA9 is labeled with Zenon^{™} Alexa Fluor^{™} 405, and p-tau217 is labeled with Zenon^{™} Alexa Fluor^{™} 488;
   2.1 1 µg of antibody in PBS (at a concentration of 0.2 µg/µL in 5 µL of PBS);
   2.2 5 µL of dye is added to and uniformly mixed with the antibody (1 µg), and the mixture is incubated at the room temperature (25°C) for 20 min in the dark;
   2.3 3 µL (3 µg) of blocking agent (an IgG blocking agent diluted to 1 µg/µL) is added to and uniformly mixed with the solution prepared in step 3.2, and the mixture is incubated at the room temperature (25°C) for 10 min in the dark;
   2.4 The above mixture is diluted with PBS (filtered with a filter of 0.22 µm) to a total volume of 50 µL;
   2.5 5 µL of Zenon^{™} Alexa Fluor^{™} 488 labeled p-tau217 is added to and uniformly mixed with the sample prepared in step (a), and the mixture is incubated at the room temperature (25°C) for 5 min in the dark;
   2.6 3 µL of Zenon^{™} Alexa Fluor^{™} 405 labeled SHISA9 is added to and uniformly mixed with the sample prepared in step 2.5, and the mixture is incubated at 4°C overnight in the dark.
3. Fixation of sample
   3.1 20 µL of 4% PFA is added to and uniformly mixed with the labeled sample, and the mixture is incubated at the room temperature (25°C) for 20 min in the dark;
   3.2 The fixed sample is diluted with 170 µL of PBS (filtered with a filter of 0.22 µm) for later use.

### (c) Detection

The intensity of a labeled signal and a particle size distribution are detected by using a NanoFCM nanoparticle flow cytometer to determine the presence and content of relevant biomarkers.

### Example 1

Plasma samples were acquired from 12 age- and sex-matched 98 subjects with Alzheimer's disease (an AD group) and healthy controls (a CT group), respectively, and an experiment was carried out by the above experimental method 1.

The number of extracellular vesicles respectively labeled with a fluorescent SHISA9 antibody only, a fluorescent p-tau217 antibody only, and both of the fluorescent SHISA9 antibody and the fluorescent p-tau217 antibody was detected by using a NanoFCM nanoparticle flow cytometer, ratios of the extracellular vesicles to total extracellular vesicles were calculated, and differences between the two groups over different particle size ranges were analyzed.

There is a significant difference (** indicates p<0.01) between the AD group and the CT group in terms of the ratio of the number of single-labeled SHISA9 protein-positive extracellular vesicles to the total numbers of extracellular vesicles in plasma with a particle size of 60-100 nm, 100-150 nm and the full particle size range (see Fig. 1A). There is an extremely significant difference (**** indicates p<0.0001) between the AD group and the CT group in terms of the ratio of the number of single-labeled p-tau217 protein-positive extracellular vesicles to the total number of extracellular vesicles in plasma with a particle size of 60-100 nm and the full particle size range, and there is a significant difference (** indicates p<0.01) between the AD group and the CT group in terms of the ratio of the number of single-labeled p-tau217 protein-positive extracellular vesicles to the total number of extracellular vesicles in plasma with a particle size of 100-150 nm (see Fig. 1B). There is an extremely significant difference (*** indicates p<0.001) between the AD group and the CT group in terms of the ratio of the number of SHISA9 protein- and p-tau217 protein-positive extracellular vesicles to the total number of extracellular vesicles in plasma with a particle size of 100-150 nm and the full particle size range (see Fig. 1C).

The diagnosis efficiency was assessed by receiver operating characteristic curve (ROC) analysis, the area under curve (AUC) of the single-labeled SHISA9 protein-positive extracellular vesicles with a particle size of 60-100 nm is 0.749, the AUC of the double-labeled SHISA9 proteinand p-tau217 protein-positive extracellular vesicles with a particle size of 100-150 nm is 0.890, and the data of SHISA9 60-100 nm and SHISA9+p-tau217 100-150 nm is entered by logistic regression analysis Enter to obtain an AUC of 0.891 (see Fig. 1D). The AUC of the single-labeled p-tau217 protein-positive extracellular vesicles with a particle size of 60-100 nm is 0.877, the AUC of the double-labeled SHISA9 protein- and p-tau217 protein-positive extracellular vesicles with a particle size of 100-150 nm is 0.890, and the data of p-tau217 60-100 nm and SHISA9+p-tau217 100-150 nm is entered by logistic regression analysis Enter to obtain an AUC of 0.910 (see Fig. 1E).

Results of Example 1 show that SHISA9 protein-positive extracellular vesicles, p-tau217 protein-positive extracellular vesicles, and SHISA9 protein- and p-tau217 protein-positive extracellular vesicles have good potential for diagnosis of Alzheimer's disease, especially the combination of p-tau217 protein-positive extracellular vesicles and SHISA9 protein- and p-tau217 protein-positive extracellular vesicles for diagnosis.

Experimental method 2:
(a) Pretreatment of peripheral body fluid: enrichment of extracellular vesicles by ultracentrifugation
   1. The plasma of a subject is rapidly dissolved in a water bath at 37°C within 1 min, centrifuged at 4°C and 2,000 g for 15 min, transferred to a new 1.5 mL centrifuge tube, and centrifuged at 4°C and 12,000 g for 30 min;
   2. 100 µL of plasma is transferred to a 1.5 mL ultracentrifuge tube and diluted with 900 µL of PBS (filtered with a filter of 0.22 µm), and the diluted plasma is centrifuged at 4°C and 100,000 g for 30 min;
   3. 800 µL of supernatant is carefully removed, an equal volume of PBS (filtered with a filter of 0.22 µm) is added to and uniformly mixed with the plasma, and the plasma is centrifuged at 4°C and 100,000 g for 30 min, a supernatant is removed until 200 µL of plasma is retained, the tube wall is repeatedly blown and beat by using a tip to resuspend and uniformly mix extracellular vesicles, and the extracellular vesicles are subpackaged and can be stored at -80°C;
   4. 20 µL of extracellular vesicles is transferred to a 100 kDa ultrafiltration tube, 200 µL of 0.1% Triton X-100/PBS is added, and the extracellular vesicles are incubated at the room temperature for 30 min;
   5. The extracellular vesicles in the ultrafiltration tube are centrifuged at 4°C and 12,000g for 3 min, 200 µL of PBS (filtered with a filter of 0.22µm) is added, and the extracellular vesicles are centrifuged;
   6. The remaining liquid in the ultrafiltration tube is transferred to a new 1.5mL centrifuge tube for later use.
(b) Labeling of antibodies for detection of biomarkers by labeling methods
   Alexa fluorescent labels are used to label an antibody targeting central nervous system-derived extracellular vesicle biomarkers and an antibody targeting Parkinson's disease-associated biomarkers. The antibody targeting central nervous system-derived extracellular vesicle biomarkers is a GLT1 monoclonal antibody, and the antibody targeting Parkinson's disease-associated biomarkers is an α-synuclein (α-syn) monoclonal antibody (syn211 or MJFR14). Labeling reagents are a GLT1 monoclonal antibody label purchased from Zenon^{™} Alexa Fluor^{™} 405 and an α-synuclein (α-syn) monoclonal antibody label purchased from Zenon^{™} Alexa Fluor^{™} 488.
   1. The GLT1 antibody is labeled using a Zenon^{™} Alexa Fluor^{™} 405 kit according to the product specification: 5 µL of fluorescent secondary antibody and 1 µg of GLT1 antibody are incubated at the room temperature for 5 min in the dark, 5 µL of IgG is added, and the mixture is incubated at the room temperature for 5 min in the dark;
   2. 0.2 µg of GLT1 antibody is added to each sample, and the sample is incubated at the room temperature for 30 min in the dark;
   3. The α-synuclein monoclonal antibody (syn211 or MJFR14) is labeled using a Zenon^{™} Alexa Fluor^{™} 488 kit according to the product specification: 5 µL of fluorescent secondary antibody and 1 µg of α-syn antibody are incubated at the room temperature for 5 min in the dark, 5 µL of IgG is added, and the mixture is incubated at the room temperature for 5 min in the dark;
   4. 0.2 µg of α-syn antibody (syn211 or MJFR14) is added to each sample, and the sample is incubated at the room temperature for 30 min in the dark;
   5. The sample is diluted with PBS (filtered with a filter of 0.22 µm) to 400 µL for later use.
(c) Detection
   The intensity of a labeled signal is detected by using an Apogee nanoparticle flow cytometer to determine the presence and content of central nervous system-derived and disease-associated biomarkers in a peripheral body fluid.

### Example 2

Plasma samples were collected from clinical cohorts (comprising 103 subjects with Parkinson's disease (a PD group), 45 subjects with multiple system atrophy (an MSA group), and 106 age- and sex-matched healthy controls (a CT group)) by the above experimental method 2 and detected by the Apogee nanoparticle flow cytometry immunofluorescence labeling technique.

GLT1 protein-positive extracellular vesicles, α-syn protein (syn211 and MJFR14 are anti-α-syn protein antibodies)-positive extracellular vesicles (see Fig. 2A) can be respectively detected by the Apogee nanoparticle flow cytometry immunofluorescence labeling technique, and meanwhile, GLT1 protein- and α-syn protein-dual positive extracellular vesicles can also be detected, so the technique has good reliability and repeatability (see Fig. 2B and Fig. 2C).

Plasma samples were collected from clinical cohorts (comprising 103 subjects with Parkinson's disease (a PD group), 45 subjects with multiple system atrophy (an MSA group), and 106 age- and sex-matched healthy controls (a CT group)) by the above the experimental method 2, and the number of GLT1 protein- and α-syn protein-positive extracellular vesicles in plasma was detected by the Apogee nanoparticle flow cytometry immunofluorescence labeling technique. In terms of the number of GLT1 protein- and α-syn protein-dual positive extracellular vesicles, the PD group is extremely significantly (*** indicates p< 0.001) greater than the MSA group or the CT group, while there is no significant difference between the MSA group and the CT group (see Fig. 3A and Fig. 3B).

The number of GLT1 protein- and α-syn protein-positive extracellular vesicles can be used for clinical diagnosis to better distinguish patients with PD from healthy controls, and patients with PD from patients with MSA, so it has the potential for clinical diagnosis of Parkinson's disease and differential diagnosis of MSA.

In CN106062559B, it is reported that L1CAM is used as a marker, central nervous system-derived exosomes (a type of extracellular vesicle, with a diameter of about 30-150 nm) are first enriched, and the relevant markers in the exosomes are detected using the Luminex detection platform.

Plasma samples of cohorts comprising 267 patients with Parkinson's disease (a PD group) and 215 age- and sex-matched normal controls (a CT group) were used for detection of relevant protein marker α-syn. There is no statistical difference between the PD group and the CT group in terms of direct detection of α-syn proteins in plasma (see Fig. 1 and Fig. 2 of CN106062559B), while in terms of the number of α-syn proteins in plasma exosomes that are captured by anti-L1CAM and the ratio of the α-syn proteins to total α-syn proteins in plasma, the PD group is statistically significantly (** indicates p<0.01) greater than the CT group (see Fig. 1 and Fig. 2 of CN106062559B), and the significant differences are equivalent to those in terms of α-syn proteins in cerebrospinal fluid (clinical cohorts comprising 100 patients with Parkinson's disease and 100 normal controls) (** indicates p<0.01) (see Fig. 1 and Fig. 2 of CN106062559B).

In order to further assess the potential of α-syn proteins in plasma exosomes that were captured by anti-L1CAM to assist clinical diagnosis of Parkinson's disease, Pearson correlation analysis and receiver operating characteristic curve (ROC) analysis were performed. It is found that the level of α-syn proteins in plasma exosomes that are captured by anti-L1CAM correlates with the UPDRS motor score and severity of Parkinson's disease (r=0.176, p=0.004, Pearson correlation) (see Fig. 3C of CN106062559B). Both the concentration of α-syn proteins in exosomes (AUC=0.654, sensitivity=70.1%, and specificity=52.9%) (see Fig. 3A of CN106062559B) and the ratio of the concentration to the total α-syn protein concentration in plasma (AUC=0.657, sensitivity=71.2%, and specificity=50.0%) can provide a moderate diagnosis efficiency, which is equivalent to that of α-syn proteins in cerebrospinal fluid (AUC=0.724, sensitivity=76.8%, specificity=53.5%) (see Fig. 3B of CN106062559B).

In the Chinese invention patent application 201810136747.8, plasma samples are collected from clinical cohorts (comprising 46 patients with multiple system atrophy (an MSA group), 49 patients with Parkinson's disease (a PD group), and 50 age- and sex-matched healthy controls (a CT group)), and the concentration of fluorescence-labeled GLT1 (EAAT2) extracellular vesicles is measured by using a Nanosight nanoparticle tracking analyzer to assess the potential of using the concentration of extracellular vesicles containing a GLT1 fluorescent label for diagnosis of PD. Receiver operating characteristic curve analysis (ROC analysis) is performed. By observation of the differential diagnosis efficiency of multiple system atrophy and Parkinson's disease, it is found that the diagnosis efficiency of Parkinson's disease is moderate (AUC=0.6750, sensitivity=66.00%, and specificity=70.45%), and the differential diagnosis efficiency of Parkinson's disease and multiple system atrophy is moderate (AUC=0.6948, sensitivity=65.91%, and specificity=78.57%) (see Fig. 3 of the patent application 201810136747.8).

As described above, compared with the reported methods, the system or the method of this application can achieve a similar PD diagnosis effect. Moreover, the method of this application is more time-saving, efficient, highly clinically applicable and has good prospects due to the simultaneous detection of two or more markers in biological fluids.

Experimental method 3:
(a) Pretreatment of peripheral body fluid: ultrafiltration centrifugation
   1. About 100 µL of plasma of a subject is thawed in a water bath at 37°C, centrifuged at 4°C and 2,000g for 15 min, transferred to a new 1.5 mL centrifuge tube, and centrifuged at 4°C and 12,000 g for 30 min;
   2. The centrifuged plasma supernatant is transferred to a new 1.5 mL centrifuge tube and uniformly mixed;
   3. 200 µL of 0.1% Triton X-100/PBS (filtered with a filter of 0.22 µm) is added to 100 kDa ultrafiltration tube, 50 µL of centrifuged plasma is added, and the plasma is incubated at the room temperature for 30 min;
   4. After being incubated, the plasma is centrifuged at 4°C and 12,000 g for 3.5 min;
   5. 200 µL of 0.1% Triton X-100/PBS (filtered with a filter of 0.22 µm) is added to the ultrafiltration tube, and the plasma is centrifuged at 4°C and 12,000 g for 3.5 min;
   6. The plasma is filtered, the remaining part (in the inner core of the ultrafiltration tube) is sucked out with a pipette, and subpackaged at a volume of 20 µL/tube which is a volume required by each reaction unit for later use.
(b) Labeling of antibodies for detection of biomarkers by labeling methods
   Reagents used:
   Alexa fluorescent labels are used to label an antibody targeting central nervous system-derived extracellular vesicle biomarkers and an antibody targeting multiple system atrophy - associated biomarkers. The antibody targeting central nervous system-derived extracellular vesicle biomarkers is a CNPase monoclonal antibody. The antibody targeting multiple system atrophyassociated biomarkers is an α-synuclein (α-syn) monoclonal antibody. Labeling reagents are a CNPase monoclonal antibody label purchased from Zenon^{™} Alexa Fluor^{™} 488 and an α-synuclein (α-syn) monoclonal antibody label purchased from Zenon^{™} Alexa Fluor^{™} 405.
   1. The CNPase antibody is labeled using a Zenon^{™} Alexa Fluor^{™} 488 kit according to the product specification: 5 µL of fluorescent secondary antibody and 1 µg of CNPase antibody are incubated at the room temperature for 15 min in the dark, 5 µL of IgG is added, and the mixture is incubated at the room temperature for 5 min in the dark;
   2. 0.2 µg of CNPase antibody is added to each sample (20 µL), and the sample is incubated at the room temperature for 30 min in the dark;
   3. The α-synuclein monoclonal antibody is labeled using a Zenon^{™} Alexa Fluor^{™} 405 kit according to the product specification: 5 µL of fluorescent secondary antibody and 1 µg of α-syn antibody are incubated at the room temperature for 15 min in the dark, 5 µL of IgG is added, and the mixture is incubated at the room temperature for 5 min in the dark;
   4. 0.2 µg of α-syn antibody is added to each sample (20 µL), and the sample is incubated at the room temperature for 30 min in the dark;
   5. The sample is diluted with PBS (filtered with a filter of 0.22 µm) to 400 µL for later use.
(c) Detection
   The intensity of a labeled signal is detected by using an Apogee nanoparticle flow cytometer to determine the presence and content of central nervous system-derived and disease-associated biomarkers in a peripheral body fluid.

### Example 3

Plasma samples of small clinical cohorts (comprising 15 subjects with Parkinson's disease (a PD group), 17 subjects with multiple system atrophy (an MSA group), and 17 age- and sex-matched healthy controls (a HC group)) were detected by the Apogee nanoparticle flow cytometry immunofluorescence labeling technology according to the above experimental method 3. It was detected that in terms of the number of CNPase protein-positive extracellular vesicles in plasma, there is no significant difference between the MSA group and the PD group, while there is a significant difference (* indicates p<0.05) between the MSA group and the HC group (see Fig. 4A). In terms of the number of CNPase protein- and α-syn protein (labeled with an anti-MJFR14 antibody)-dual positive extracellular vesicles, there is no significant difference between the MSA group and the PD group, and the MSA group and the HC group (see Fig. 4B). In terms of the number of CNPase proteinand α-syn protein-dual positive extracellular vesicles in plasma, after it is normalized by the total number of extracellular vesicles in plasma , there is a significant difference (* indicates p<0.05) between the MSA group and the HC group, and the MSA group and the PD group (see Fig. 4C).

The number of CNPase protein- and α-syn protein-dual positive extracellular vesicles has the potential for diagnosis of multiple system atrophy (MSA) and differential diagnosis of multiple system atrophy and Parkinson's disease (PD).

The above are preferred embodiments of the present application only and are not intended to limit the present application in other forms. Those skilled in the art may make modifications or transformations to the above disclosed technical content to obtain equivalent embodiments of equivalent changes. However, any simple amendment, equivalent change, and transformation made to the above embodiments without departing from the content of the technical solutions of the present application according to the technical essence of the present application shall fall within the scope of protection of the technical solutions of the present application.

## Claims

1. A method for diagnosing a central nervous system disease, comprising:
detecting a central nervous system-derived marker in a biological fluid of a subject,
detecting a central nervous system disease-associated marker in the biological fluid of the subject, and
diagnosing whether the subject suffers from a central nervous system disease based on detection results of the central nervous system-derived marker and the central nervous system disease-associated marker.

2. The method according to claim 1, wherein
the detecting a central nervous system-derived marker in a biological fluid of a subject and the detecting a central nervous system disease-associated marker in the biological fluid of the subject are performed simultaneously.

3. The method according to claim 1 or 2, further comprising:
acquiring the biological fluid of the subject, and pretreating the biological fluid to enrich biological samples in the biological fluid,
the detecting a central nervous system-derived marker in a biological fluid of a subject referring to detecting a central nervous system-derived marker in enriched biological samples of the subject, and
the detecting a central nervous system disease-associated marker in the biological fluid of the subject referring to detecting a central nervous system disease-associated marker in the enriched biological samples of the subject.

4. The method according to any one of claims 1 to 3, further comprising:
counting biological samples in which both of the central nervous system-derived marker and the central nervous system disease-associated marker are detected, and/or
measuring particle sizes of the biological samples in which both of the central nervous system-derived marker and the central nervous system disease-associated marker are detected, and
diagnosing whether the subject suffers from a central nervous system disease based on the number of the biological samples and/or the particle sizes of the biological samples.

5. The method according to claim 3 or 4, wherein
the biological samples are extracellular vesicles, and
preferably, the extracellular vesicles are neuron-derived extracellular vesicles, astrocyte-derived extracellular vesicles, oligodendrocyte-derived extracellular vesicles or microglia-derived extracellular vesicles.

6. The method according to any one of claims 1 to 5, wherein the biological fluid is selected from one or more of blood, serum, plasma, saliva, urine, lymph, semen or milk.

7. The method according to any one of claims 1 to 6, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.

8. The method according to claim 7, wherein
the cerebrovascular diseases comprise cerebral infarction, cerebral hemorrhage, cerebral blood supply insufficiency, and transient ischemic attack;
the spinal cord lesions comprise acute myelitis, polio, and syringomyelia;
the central nervous system infections comprise encephalitis and meningitis;
the inherited diseases of the nervous system comprise neurocutaneous syndrome and spinocerebellar ataxia;
the dysplastic diseases of the nervous system comprise congenital hydrocephalus and cerebral palsy;
the autonomic nervous system disease is autonomic nervous dysfunction; and
the degenerative diseases of the nervous system comprise degenerative dementia, dyskinetic diseases, and prion diseases.

9. The method according to claim 8, wherein
the degenerative dementia comprises Alzheimer's disease (AD), frontotemporal dementia (FTD), Parkinson's disease dementia (PDD), and dementia with Lewy bodies (DLB); and
the dyskinetic diseases comprise motor neuron disease (MND), Parkinson's disease (PD), Huntington's disease (HD), Sydenham's chorea, Wilson's disease (WD), multiple system atrophy (MSA), progressive supranuclear palsy (PSP), and corticobasal degeneration (CBD).

10. The method according to any one of claims 1 to 9, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.

11. The method according to any one of claims 1 to 10, wherein the central nervous system-derived markers and/or the central nervous system disease-associated markers are each selected from one or two or three of proteins, nucleic acids, and lipids.

12. The method according to claim 10 or 11, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE),
preferably, the central nervous system-derived markers are markers located on the surface of central nervous system-derived extracellular vesicles, and
further preferably, the central nervous system-derived markers are selected from one or more of L1CAM, SHISA9, CNPase, and GLT1 (EAAT2).

13. The method according to claim 11, wherein the central nervous system disease-associated markers are proteins selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.

14. The method according to claim 11, wherein the central nervous system disease-associated markers are nucleic acids selected from one or more of DNA or RNA serving as biomarkers associated with Alzheimer's disease (AD), and DNA or RNA serving as biomarkers associated with Parkinson's disease (PD) and Parkinson's syndromes (e.g., multiple system atrophy (MSA), progressive supranuclear palsy (PSP), corticobasal degeneration (CBD), and dementia with Lewy bodies (DLB)).

15. The method according to claim 14, wherein the DNA or RNA serving as biomarkers associated with AD comprises DNA or RNA associated with genes UBAP2L, YBX1, SERF2, UBE2B, RPL10A, H3F3AP4, PPP2CA, NMD3, RNF7, RPLPO, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, or GREB 1.

16. The method according to claim 14, wherein the DNA or RNA serving as biomarkers associated with PD comprises DNA or RNA associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC 100132832, or BLOC 1S 1-RDH5.

17. The method according to claim 3, wherein the method for acquiring the biological fluid of the subject and pretreating the biological fluid to enrich biological samples is selected from one or more of centrifugation, ultracentrifugation, ultrafiltration tube filtration, polymeric sedimentation, and specific antibody capture.

18. The method according to claim 11, wherein the central nervous system-derived markers are proteins, and the step of detecting a central nervous system-derived marker in a biological fluid of a subject comprises: reacting the biological fluid of the subject, preferably extracellular vesicles of the subject, with a labeled antibody that specifically reacts with central nervous system-derived markers, and detecting the intensity of a labeled signal after the reaction to determine the presence and content of the central nervous system-derived markers.

19. The method according to claim 11, wherein the central nervous system disease-associated markers are proteins, and the step of detecting a central nervous system disease-associated marker in the biological fluid of the subject comprises: reacting the biological fluid of the subject, preferably extracellular vesicles of the subject, with a labeled antibody that specifically reacts with central nervous system disease-associated markers, and detecting the intensity of a labeled signal after the reaction to determine the presence and content of the central nervous system disease-associated markers.

20. The method according to claim 18 or 19, wherein the labeling is selected from one or more of fluorescence labeling, isotope labeling, enzyme labeling, chemiluminescence labeling, quantum dot labeling, and colloidal gold labeling.

21. The method according to claim 11, wherein the central nervous system-derived markers are nucleic acids, and the step of detecting a central nervous system-derived marker in a biological fluid of a subject comprises: lysing the biological fluid of the subject, preferably extracellular vesicles of the subject, and detecting the presence and content of central nervous system-derived nucleic acids by a DNA or RNA probe or sequencing technique.

22. The method according to claim 11, wherein the central nervous system disease-associated markers are nucleic acids, and the step of detecting a central nervous system disease-associated marker in the biological fluid of the subject comprises: lysing the biological fluid of the subject, preferably extracellular vesicles of the subject, and detecting the presence and content of central nervous system disease-associated nucleic acids by a DNA or RNA probe or sequencing technique.

23. A system for diagnosing a central nervous system disease, comprising:
a first detection module, configured to detect a central nervous system-derived marker in a biological fluid of a subject,
a second detection module, configured to detect a central nervous system disease-associated marker in the biological fluid of the subject, and
a diagnosis module, configured to diagnose whether the subject suffers from a central nervous system disease based on detection results of the central nervous system-derived marker and the central nervous system disease-associated marker respectively obtained by the first detection module and the second detection module.

24. The system according to claim 23, wherein
the first detection module and the second detection module simultaneously detect the central nervous system-derived marker and the central nervous system disease-associated marker in the biological fluid of the subject; or
the first detection module and the second detection module are the same module configured to simultaneously detect the central nervous system-derived marker and the central nervous system disease-associated marker in the biological fluid of the subject.

25. The system according to claim 23 or 24, further comprising:
an enrichment module, configured to acquire the biological fluid of the subject and pretreat the biological fluid to enrich biological samples in the biological fluid,
wherein the first detection module and the second detection module detect the central nervous system-derived marker and the central nervous system disease-associated marker in enriched biological samples of the subject.

26. The system according to any one of claims 23 to 25, further comprising:
a counting module, configured to count biological samples in which both of the central nervous system-derived marker and the central nervous system disease-associated marker are detected, and/or
a particle size measurement module, configured to measure particle sizes of the biological samples in which both of the central nervous system-derived marker and the central nervous system disease-associated marker are detected, and
the diagnosis module, configured to diagnose whether the subject suffers from a central nervous system disease based on the number of the biological samples and/or the particle sizes of the biological samples.

27. The system according to claim 25 or 26, wherein
the biological samples are extracellular vesicles, and
preferably, the extracellular vesicles are neuron-derived extracellular vesicles, astrocyte-derived extracellular vesicles, oligodendrocyte-derived extracellular vesicles or microglia-derived extracellular vesicles.

28. The system according to any one of claims 23 to 27, wherein the biological fluid is selected from one or more of blood, serum, plasma, saliva, urine, lymph, semen, and milk.

29. The system according to any one of claims 23 to 28, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.

30. The system according to claim 29, wherein
the cerebrovascular diseases comprise cerebral infarction, cerebral hemorrhage, cerebral blood supply insufficiency, and transient ischemic attack;
the spinal cord lesions comprise acute myelitis, polio, and syringomyelia;
the central nervous system infections comprise encephalitis and meningitis;
the inherited diseases of the nervous system comprise neurocutaneous syndrome and spinocerebellar ataxia;
the dysplastic diseases of the nervous system comprise congenital hydrocephalus and cerebral palsy;
the autonomic nervous system disease is autonomic nervous dysfunction; and
the degenerative diseases of the nervous system comprise degenerative dementia, dyskinetic diseases, and prion diseases.

31. The system according to claim 30, wherein
the degenerative dementia comprises Alzheimer's disease (AD), frontotemporal dementia (FTD), Parkinson's disease dementia (PDD), and dementia with Lewy bodies (DLB); and
the dyskinetic diseases comprise motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).

32. The system according to any one of claims 23 to 31, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.

33. The system according to any one of claims 23 to 32, wherein the central nervous system-derived markers and/or the central nervous system disease-associated markers are each selected from one or two or three of proteins, nucleic acids, and lipids.

34. The system according to claim 32 or 33, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), acetylcholine transporters (vesicular acetylcholine transporter (VAChT), and acetylcholinesterase (AchE),
preferably, the central nervous system-derived markers are markers located on the surface of central nervous system-derived extracellular vesicles, and
further preferably, the central nervous system-derived markers are selected from one or more of L1CAM, SHISA9, CNPase, and GLT1 (EAAT2).

35. The system according to claim 34, wherein the central nervous system disease-associated markers are proteins selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.

36. The system according to claim 34, wherein the central nervous system disease-associated markers are nucleic acids selected from one or more of DNA or RNA serving as biomarkers associated with Alzheimer's disease (AD), and DNA or RNA serving as biomarkers associated with Parkinson's disease (PD) and Parkinson's syndromes (e.g., multiple system atrophy (MSA), progressive supranuclear palsy (PSP), corticobasal degeneration (CBD), and dementia with Lewy bodies (DLB)).

37. The system according to claim 36, wherein the DNA or RNA serving as biomarkers associated with AD comprises DNA or RNA associated with genes UBAP2L, YBX1, SERF2, UBE2B, RPL10A, H3F3AP4, PPP2CA, NMD3, RNF7, RPLPO, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, or GREB 1.

38. The system according to claim 36, wherein the DNA or RNA serving as biomarkers associated with PD comprises DNA or RNA associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC 100132832, or BLOC 1S 1-RDH5.

39. The system according to claim 25, wherein the enrichment module comprises a sub-module configured to perform one or more of the following steps: centrifugation, ultracentrifugation, ultrafiltration tube filtration, polymeric sedimentation, and specific antibody capture.

40. The system according to claim 33, wherein the central nervous system-derived markers are proteins, and the first detection module is configured to react the biological fluid of the subject, preferably extracellular vesicles of the subject, with a labeled antibody that specifically reacts with central nervous system-derived markers, and detect the intensity of a labeled signal after the reaction to determine the presence and content of the central nervous system-derived markers.

41. The system according to claim 33, wherein the central nervous system disease-associated markers are proteins, and the second detection module is configured to react the biological fluid of the subject, preferably extracellular vesicles of the subject, with a labeled antibody that specifically reacts with central nervous system disease-associated markers, and detect the intensity of a labeled signal after the reaction to determine the presence and content of the central nervous system disease-associated markers.

42. The system according to claim 40 or 41, wherein the labeling is selected from one or more of fluorescence labeling, isotope labeling, enzyme labeling, chemiluminescence labeling, quantum dot labeling, and colloidal gold labeling.

43. The system according to claim 33, wherein the central nervous system-derived markers are nucleic acids, and the first detection module is configured to lyse the biological fluid of the subject, preferably extracellular vesicles of the subject, and detect the presence and content of central nervous system-derived nucleic acids by a DNA or RNA probe or sequencing technique.

44. The system according to claim 33, wherein the central nervous system disease-associated markers are nucleic acids, and the second detection module is configured to lyse the biological fluid of the subject, preferably extracellular vesicles of the subject, and detect the presence and content of central nervous system disease-associated nucleic acids by a DNA or RNA probe or sequencing technique.

45. A composition for detecting a central nervous system disease, comprising:
an antibody, used for targeting a central nervous system-derived marker, and
an antibody, used for targeting a central nervous system disease-associated marker.

46. The composition according to claim 45, wherein the central nervous system disease is selected from one or more of cerebrovascular diseases, spinal cord lesions, central nervous system infections, inherited diseases of the nervous system, dysplastic diseases of the nervous system, autonomic nervous system diseases, demyelinating diseases of the nervous system, epilepsy, and degenerative diseases of the nervous system.

47. The composition according to claim 46, wherein
the cerebrovascular diseases comprise cerebral infarction, cerebral hemorrhage, cerebral blood supply insufficiency, and transient ischemic attack;
the spinal cord lesions comprise acute myelitis, polio, and syringomyelia;
the central nervous system infections comprise encephalitis and meningitis;
the inherited diseases of the nervous system comprise neurocutaneous syndrome and spinocerebellar ataxia;
the dysplastic diseases of the nervous system comprise congenital hydrocephalus and cerebral palsy;
the autonomic nervous system disease is autonomic nervous dysfunction; and
the degenerative diseases of the nervous system comprise degenerative dementia, dyskinetic diseases, and prion diseases.

48. The composition according to claim 47, wherein
the degenerative dementia comprises Alzheimer's disease (AD), frontotemporal dementia (FTD), Parkinson's disease dementia (PDD), and dementia with Lewy bodies (DLB); and
the dyskinetic diseases comprise motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).

49. The composition according to any one of claims 45 to 48, wherein the central nervous system-derived markers are biomarkers derived from the following neurons or cells: mature neurons, oligodendrocytes, astrocytes, microglia, immature neurons, Schwann cells, radial glia, intermediate progenitors, glutamatergic neurons, GABAergic neurons, dopaminergic neurons, serotonergic neurons, and cholinergic neurons.

50. The composition according to any one of claims 45 to 49, wherein the central nervous system-derived markers are selected from one or more of G protein-coupled receptor 162 (GPR162), hyperpolarization activated cyclic nucleotide-gated potassium channel 1 (HCN1), gamma-aminobutyric acid type A receptor subunit delta (GABRD), neuroligin 3 (NLGN3), SHISA9, contactin-1 (CNTN1), NeuN, MAP2, neurofilament light (NF-L), neurofilament medium (NF-M), neurofilament heavy (NF-H), synaptophysin, syntaxin, PSD95, L1CAM, doublecortin, beta III tubulin, NeuroD1, TBR1, stathmin 1, MPZ, NCAM, GAP43, S100, CNPase, olig 1, olig 2, olig 3, MBP, OSP, MOG, SOX10, NG2, GFAP, GLAST (EAAT1), GLT1 (EAAT2), glutamine synthetase, S100-β, ALDH1L1, CD11b, CD45, Iba1, F4/80, CD68, CD40, vimentin, nestin, PAX6, HES1, HES5, GFAP, GLAST, BLBP, TN-C, N-cadherin, SOX2, TBR2, MASH1 (Ascl1), vGluT1, vGluT2, NMDAR, glutaminase, glutamine synthetase, GABA transporter 1 (GAT1), GABA_{B} receptor 1, GABA_{B} receptor 2, GAD65, GAD67, tyrosine hydroxylase (TH), dopamine transporter (DAT), FOXA2, GIRK2, Nurr1, LMX1B, tryptophan hydroxylase (TPH), serotonin transporter, Petl, choline acetyl transferase (ChAT), vesicular acetylcholine transporter (VAChT), and acetylcholinesterase (AchE),
preferably, the central nervous system-derived markers are markers located on the surface of central nervous system-derived extracellular vesicles, and
further preferably, the central nervous system-derived markers are selected from one or more of L1CAM, SHISA9, CNPase, and GLT1 (EAAT2).

51. The composition according to any one of claims 45 to 50, wherein the central nervous system disease-associated markers are selected from one or more of Aβ1-40 (Aβ40) protein, Aβ1-42 (Aβ42) protein, oligomeric Aβ (oligo-Aβ) protein, tau protein, phosphorylated tau (p-tau) protein, α-synuclein, phosphorylated α-synuclein at position S129 (pS129), oligomeric α-synuclein (oligo-α-syn), and prion protein.

52. The composition according to any one of claims 45 to 51, wherein the antibody used for targeting central nervous system-derived markers and the antibody used for targeting central nervous system disease-associated markers are labeled antibodies, and preferably, the labeling is selected from one or more of fluorescence labeling, isotope labeling, enzyme labeling, chemiluminescence labeling, quantum dot labeling, and colloidal gold labeling.

53. A kit for detecting a central nervous system disease in a subject, comprising:
a reagent, used for detecting a central nervous system-derived marker in a biological fluid of a subject, and
a reagent, used for detecting a central nervous system disease-associated marker in the biological fluid of the subject.

54. The kit according to claim 53, wherein the reagent used for detecting a central nervous system-derived marker in a biological fluid of a subject and the reagent used for detecting a central nervous system disease-associated marker in the biological fluid of the subject are the composition according to any one of claims 45 to 52.

55. The kit according to claim 53, wherein
the reagent used for detecting a central nervous system-derived marker in a biological fluid of a subject is a primer or a probe targeting the central nervous system-derived marker, and
the reagent used for detecting a central nervous system disease-associated marker in the biological fluid of the subject is a primer or a probe targeting the central nervous system disease-associated marker.

56. The kit according to claim 53, further comprising:
a reagent and an apparatus, used for acquiring the biological sample of the subject, and preferably, a reagent and an apparatus, used for acquiring extracellular vesicles of the subject.

57. The kit according to claim 53, wherein the central nervous system disease is selected from one or more of cerebrovascular diseases, spinal cord lesions, central nervous system infections, inherited diseases of the nervous system, dysplastic diseases of the nervous system, autonomic nervous system disease, demyelinating diseases of the nervous system, epilepsy, and degenerative diseases of the nervous system.

58. The kit according to claim 57, wherein
the cerebrovascular diseases comprise cerebral infarction, cerebral hemorrhage, cerebral blood supply insufficiency, and transient ischemic attack;
the spinal cord lesions comprise acute myelitis, polio, and syringomyelia;
the central nervous system infections comprise encephalitis and meningitis;
the inherited diseases of the nervous system comprise neurocutaneous syndrome and spinocerebellar ataxia;
the dysplastic diseases of the nervous system comprise congenital hydrocephalus and cerebral palsy;
the autonomic nervous system disease is autonomic nervous dysfunction; and
the degenerative diseases of the nervous system comprise degenerative dementia, dyskinetic diseases, and prion diseases.

59. The kit according to claim 58, wherein
the degenerative dementia comprises Alzheimer's disease (AD), frontotemporal dementia (FTD), Parkinson's disease dementia (PDD), and dementia with Lewy bodies (DLB); and
the dyskinetic diseases comprise motor neuron diseases (MNDs), Parkinson's disease (PD), Huntington's disease (HD), Sydenham's chorea, Wilson's disease (WD), multiple system atrophy (MSA), progressive supranuclear palsy (PSP), and corticobasal degeneration (CBD).
